# EUROPEAN PATENT APPLICATION

(11) **EP 2 243 842 A1**
(43) Date of publication of application: **27.10.2010**
(21) Application number: 09005576.5
(22) Date of filing: 21.04.2009
(51) Int. Cl.: C12Q 1/68

(54) **DCLK1 and cognitive ability and cognitive performance**

(71) Applicant: Bergen Teknologieverforing AS, 5006 Bergen (NO)
(72) Inventor: Le Hellard, Stephanie, 5238 Rådal (NO); Steen, Vidar Martin, 5238 Rådal (NO)
(74) Representative: Kröncke, Rolf

(57) **Abstract**

Generally, the present invention relates to the finding that the DCLK1 gene is involved in cognitive abilities and/or cognitive performance in an individual. Particularly, the present invention relates to the use of SNPs in the DCLK1 gene for determining cognitive abilities and/or cognitive performance in an individual. In a further aspect, the present invention relates to a method for testing and/or determining the cognitive abilities and/or cognitive performance of an individual. Moreover the use of compounds for improving cognitive abilities and/or cognitive performance in an individual whereby said compounds are able to increase the expression of the DCLK1 gene product is described.

## Description

Generally, the present invention relates to the finding that the DCLK1 gene is involved in cognitive abilities and/or cognitive performance in an individual. Particularly, the present invention relates to the use of SNPs in the DCLK1 gene for determining cognitive abilities and/or cognitive performance in an individual. In a further aspect, the present invention relates to a method for testing and/or determining the cognitive abilities and/or cognitive performance of an individual. Moreover the use of compounds for improving cognitive abilities and/or cognitive performance in an individual whereby said compounds are able to increase the expression of the DCLK1 gene product is described. Finally, the present invention provides methods for screening and identifying compounds altering DCLK1 acitivity and, thus, providing drugs against cognitive deficits and/or decline

### State of the Art

Empirical evidence for genetic factors underlying cognitive variation is strong, but identification of specific genetic variants has proved challenging. The study of differences in human mental abilities, measured with psychometric tools, has defined a hierarchical structure of human cognition. General cognitive ability (g) stands at the pinnacle, accounting for about 50% of the total variance in test performances. Correlated with g, there are additional, separable group factors representing distinct cognitive domains, with memory as a prominent example. Behavioral studies suggest that the impact of genetic factors on specific cognitive domains, e.g. memory, derives from both inherited contributions to general cognitive ability *and* from genetic variation that more selectively influences memory. This heritability is thought to be polygenic but, to date, molecular genetic studies have provided replicated genetic associations with cognitive function (Green AE, et al. Nat Rev Neurosc 2008 Sep; 9(9): 710-720).

Variations in the gene for the brain-derived neurotrophic factor (*BDNF*) have been examined for associations between cognitive traits and psychiatric disorders. Several studies have identified association of a single nucleotide polymorphism (SNP - rs6265) - which corresponds to an amino acid change (Val66Met) - and verbal memory, general cognitive ability, age related change in reasoning skills and hippocampal functions, but others have failed to replicate these findings. The same Val66Met genetic variant of BDNF has been largely studied as a risk factor for bipolar affective disorder, schizophrenia and other psychiatric disorders (for review see (Craddock N, Sklar P. Trends Genet 2009 Feb; 25(2): 99-105; Petryshen TL, et al. Mol Psychiatry 2009, doi:10.1038/mp.2009.1024. Since several of the cognitive traits associated with BDNF variants have been reported potentially deficient in major psychosis (e.g. verbal memory and general cognition in bipolar disorder and/or schizophrenia, these common 5 associations might reflect genetic associations to clinical endophenotypes of these disorders. Indeed, neurocognitive traits have been proposed as endophenotypes for psychosis that, because they are less clinically heterogeneous and have high heritability, would be more powerful in identifying genetic factors of susceptibility (Glahn DC, et al. Bipolar Disord 2004 Jun; 6(3): 171-182.

In view of the above, there is an ongoing need for providing means allowing the determination of cognitive abilities and/or cognitive performance in an individual.

### Brief description of the present invention

The present inventors found that variants in the DCLK1 gene are associated with memory and general cognitive abilities and cognitive performance. Thus, in a the first aspect, the present invention relates to the use of SNPs present in the DCLK1 gene according to Seq. ID No. 1 for determining cognitive abilities and/or cognitive performance in an individual.

In a second aspect the present invention relates to a method for testing and/or determining the cognitive abilities and/or cognitive performance of an individual comprising the step of identifying the presence or absence of SNPs in the DCLK1 gene according to Seq. ID No. 1 in an individual and determining the cognitive abilities and/or cognitive performance based on the presence or absence of SNP.

In another aspect, a system for determining the cognitive abilities and/or cognitive performance of an individual is provided.

Moreover, the present invention provides the use of compounds able to increase the expression of the DCLK1 gene product for improving cognitive abilities and/or cognitive performance in an individual.

Finally, the present invention relates to methods for screening and optionally identifying compounds altering DCLK1 activity and, thus, are useful for the prophylaxis or treatment of deficits or decline of cognitive abilities or cognitive performance.

### Brief description of the drawings

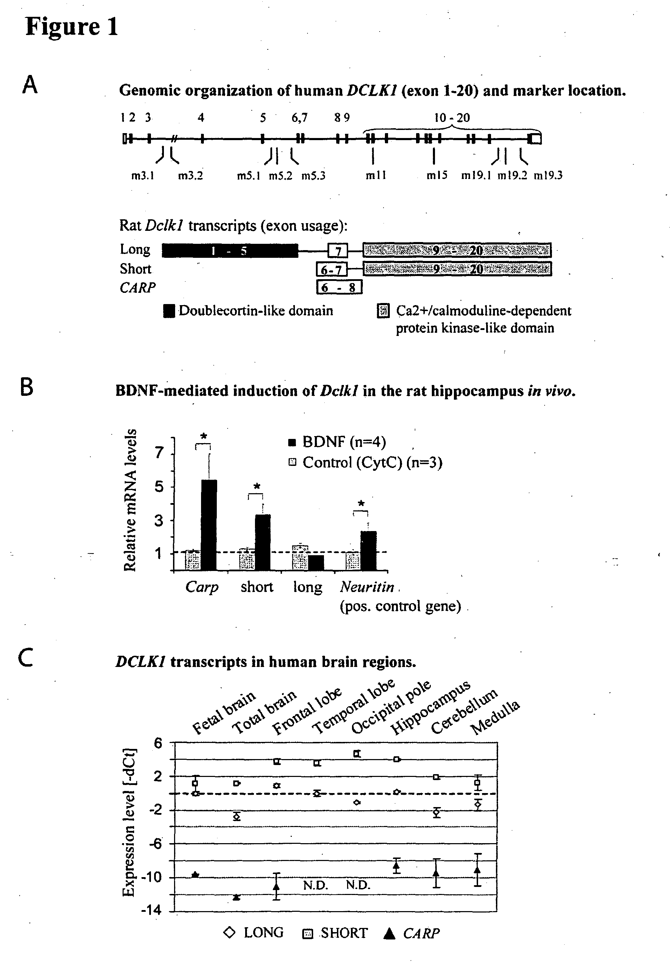

### Figure 1: Genomic organization of human DCLK1 and expression of transcript variants in human brain regions and in rat hippocampus in response to BDNF.

(A) Proposed genomic organization, exon usage and marker location for the human *DCLK1* gene. In rodents, *Dclkl* contains 20 exons producing several transcripts, such as long, short and *Carp* mRNAs. In human, the reference sequence only lists 18 exons encoding the long *DCLK1* transcript (NM_004734). With transcript-specific RT-PCR assays (see below, panel C), we show that short *DCLK1* and *CARP* are expressed in humans (with inclusion of exon 6 and 8), thus the human genomic sequence should contain 20 exons. Black, white and grey boxes illustrate protein domains encoded by different exons. The genomic locations of markers with positive scores or interactions are marked. (B) The expression of *Dclk1* variants in response to infusion of exogenous BDNF into the dentate gyrus *in vivo* were analyzed by real-time RT PCR. BDNF mediates the expression of short *Dclk1* variants and *Carp,* while the long (fulllength) *Dclk1* transcripts are unaffected (or slightly reduced). Infusion of Cytochrome C was used as a negative control. (C) RT-PCR amplification of N-terminal-("long") and C-terminal ("short") domain *DCLK1* transcripts and *CARP* expressed in human brain regions. -dCt values are given relative to the Ct of long *DCLK1* in fetal brain (dotted line, Ct=22.9, mean ± S.E.M.). N.D.: not detected / variable detection.

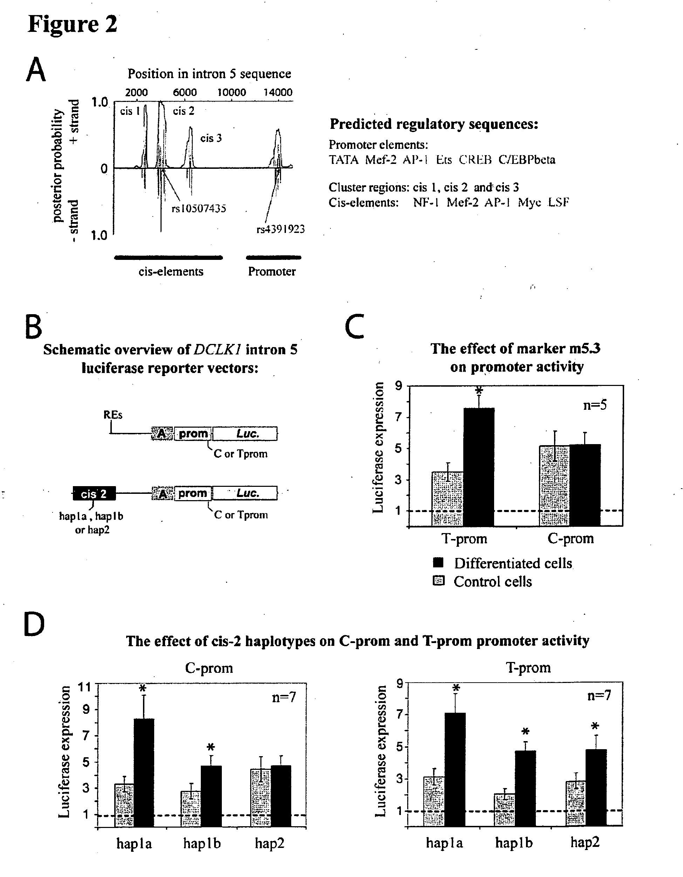

### Figure 2: In silico prediction and functional characterization of promoter elements in intron 5 of human DCLK1.

(A) Illustration of probability scores for transcription factor binding-sites in three clusters and a promoter proximal region (merged pictures). Arrows indicate the position of SNP markers. (B) Schematic representation of luciferase reporter vectors. Abbreviations: Prom, promoter; luc, luciferase reporter gene. (C) Luciferase reporter assay demonstrating basal promoter activity and allelespecific responses to neuronal differentiation of SH-SY5Y cells. (D) Luciferase reporter assays demonstrating cis2 haplotype-specifc effects in response to neuronal differentiation of SH-SY5Y cells. Luciferase signal intensities obtained from nondifferentiated cells (grey bars) and RA/BDNF-exposed cells (black bars) are compared for each reporter plasmid. Values are given as mean ± S.E.M. * Statistically significant different expression in differentiated cells as compared to control cells (ttest, p <0.05).

### Detailed description of the present invention

In a first aspect, the present invention relates to the use of SNPs present in the DCLK1 gene according to Seq. ID No. 1 for determining cognitive abilities and/or cognitive performance in an individual.

That is, the present inventers found that SNPs (single nucleotide polymorphisms) present in the DCLK1 gene allow to determine cognitive abilities and/or cognitive performance in an individual. The cognitive abilities and/or cognitive performance are for example memory, learning, but also schizophrenia, hyperactivity or bipolar affective disorder.

It was recognized that DCLK1 gene (doublecortin-like kinase 1 or doublecortin-and calmodulin kinase-like 1 gene, DCAMKL1) ande gene product are upregulated during BDNF-induced synaptic consolidation in hippocampus. Furthermore, genetic variants of the DCLK gene represent useful markers being associated with general cognition e.g. verbal memory function. The associated variants are located in potential alternative promoter and cis-regulatory elements of the DCLK1 gene and they affect BDNF-mediated expression in particular of the short forms of the DCLK1 transcripts. Thus, the present inventors found that DCLK1 is involved in cognitive performance in humans.

Typically, the cognitive abilities and/or cognitive performance are determined in human individuals. However, it is clear that also in other mammals the cognitive abilities and/or cognitive performance may be determined based on the presence of SNPs in the DCLK1 gene.

The term cognitive abilities and/or cognitive performance includes e.g. memory function, learning abilities, but also diseases associated with cognitive performance or cognitive abilities, like schizophrenia, hyperactivity, bipolar affective disorder.

In a further aspect, the present invention relates to a method for testing and/or determining the cognitive abilities and/or cognitive performance of an individual comprising the step of
- identifying the presence or absence of SNPs in the DCLK1 gene according to Seq. ID No. 1 in an individual and
- determining the cognitive abilities and/or cognitive performance based on the presence or absence of the SNP exam in the step before.

It was recognized that when specific SNPs in the DCLK1 gene are present, e. g. SNPs shown in tables 1 and 2 below, this influences the cognitive performance and cognitive abilities of individuals, e. g. reducing the memory ability or learning ability. Further, it was found that the presence of specific SNPs in the DCLK1 gene is related to schizophrenia, hyperactivity or bipolar affective disorder.

The presence or absence of SNPs may be conducted by known methods, e. g. SNPs are detected using PCR-methods or other molecular biology methods known in the art. The skilled person is well aware of suitable methods.

Based on the presence or absence of the SNPs identified by known techniques, the cognitive abilities and/or cognitive performance of said individual may be determined based on a relationship between SNP and cognitive performance or cognitive ability.

Furthermore, a system is provided for determining the cognitive ability and/or cognitive performance of an individual. Said system comprises means for identifying SNPs in the DCLK1 gene of Seq. ID No. 1 as well as instructions to assign said SNPs identified in the DCLK1 gene with the cognitive ability and/or cognitive performance. Suitable means for identifying SNPs are known in the art, e. g., said means comprises PCR-based sequence analysis etc.

Moreover, the present invention relates to the use of compounds able to increase the transcription and/or expression of the DCLK1 gene or DCLK1 gene product for improving cognitive abilities and/or cognitive performance in an individual.

The present inventors recognized that expression of DCLK1 as well as variations in DCLK1 are connected with the ability of memory, learning and other cognitive abilities and/or cognitive performances.

The association of DCLK expression on nucleotide level or peptide level with e. g. memory has not been described before.

In preferred embodiments of the present invention, the SNP to be identified or determined is at least one SNP as shown in table 1 or table 2 below.

Further, the instructions to assign the SNP present in a DCLK1 gene with a cognitive ability and/or cognitive performance of the system according to the present invention comprises preferably the tables 1 or 2 given below.

For determining an association with schizophrenia, preferably, the SNP rs7989807 is analysed. It has been found that an increased risk to deveöop schizophrenia when said SNP is present.

In another asepct, the present invention provides a method for screening compounds for preventing or treating cognitive deficits or cognitive decline, e.g. age-based cognitive decline, comprising the steps of
a) providing candidate compounds to be tested,
b) providing a cell system allowing determination of DCLK1 expression and/or activity on nulceic acid or peptide level,
c) incubating the cell system of b) with the candicate compounds to be tested, and
d) determining altered expression and/or activity of DCLK1.

Thus, the present invention relates to a method for screening, designing, engineering or otherwise producing a prophylactic or therapeutic agent for use in prophylaxis or treatment of cognitive abilities or cognitive performance whereby said agent can selectively modulate the expression or activity of DCLK1 or nucleic acids encoding the protein.

According to the present invention, candidate compounds are preferably selected from the group of isolated nucleic acids, proteins uncluding polypeptides or oligopeptides, or small molecules.

For example, the candidate compound is an siloated nucleic acid, like DNA or RNA or modified DNA or RNA molecules with modifications known in ht eart. For example, the nucleic acid molecule is a DNA oligonucleotide or a modified version, silencer RNA, interfering RNA, antisense RNA artificial microRNA, ribozyme, etc.

Further, the candidate compound may a a small molecule. In this context, the term "small molecule" particularly refers ro small organic molecules. Typically said small molecules are part of screening libraries comprising chemical, typically organical, synthetic compounds. O course, naturally occuring, isolated molecules may be screened. Suitable molecules may be designed or engineered with computer assisted programs.

Compounds useful to increase the transcription or expression of the DCLK1 gene or DCLK1 gene product are for example compounds like BDNF (brain-derived neutrophic factor) or compounds increasing BDNF expression.

Thus, DCLK1 gene and gene product is a new target suitable for the development of drugs against cognitive deficits and/or decline.

The present inventors found that SNPs of DCLK1 gene associated with cognitive ability and/or cognitive performance are located e. g. in potential alternative promote and sys-regulatory elements of DCLK1 and can affect BDNF-mediated expression of short forms of DCLK1 transcripts as shown below. As demonstrated herein, the DCLK1 gene product may be present in different forms, namely long forms, short forms or as so called Carp forms, further details are given below.

Useful screening methods for identifying suitable compounds able to modulate, e.g. to increase or decrease, the transcription or expression of DCLK1 gene or DCLK1 gene products, respectively, are known by the skilled person.

Finally, the present invention provides a method for identifying compounds useful as pharmaceuticals for the prophylaxis or treatment of cognitive deficits or cognitive decline. Further, these compounds are useful for increasing or stabilising cognitive abilities or cognitive performance. Said method encompasses the step of determining the ability of a candidate compound to alter the expression or activity of DCLK1.

The cognitive abilities and/or cognitive performance according to the present invention are preferably selected from memory ability, learning ability, or diseases like schizophrenia, hyperactivity, bipolar affective disorder and the like. For example, the SNPs are associated with the age decline of memory or learning ability of individuals as demonstrated below.

The present invention will be illustrated further by the examples given below without delimiting the present invention thereto.

### Examples

### MATERIALS AND METHODS

### 1. Samples descriptions

**The Norwegian Cognitive NeuroGenetics (NCNG) sample consists of 271** participants aged 46-75 years (mean age of 62.6 years, s.d. 7.9) that were recruited through advertisements in local newspapers in the Oslo and Bergen urban areas. Candidates were first interviewed by phone according to a check list about health and previous illness or injuries. Exclusion criteria were previously diagnosed neurological or psychiatric illness, any other chronic illness that might influence test performance, or sensory or motor impairments. Participants with a history of alcohol or substance abuse, or current addictive disorders, were also excluded. Participants had to be native speakers of Norwegian and have completed obligatory basic education (7 years for this age group) without diagnosed reading or learning disorders. Persons on adequate medication for hypertension, diabetes or hypercholesterolemia were not excluded. Participants were not allowed to consume nicotine or caffeine during the test period or in the lab premises, but were not required to abstain from these substances prior to attendance. There were 195 females (72 %) and the average total years of education was 13.9 (s.d. 3.0). The Vocabulary and Matrix Reasoning subscales of the Wechsler Abbreviated Scale of Intelligence (WASI) were used to estimate IQ, and the California Verbal Learning Test II (CVLT-II) to test verbal episodic memory.

**The Lothian Birth Cohort 1921 (LBC1921)** are surviving participants of the Scottish Mental Survey of 1932 (SMS1932). They were recruited in Edinburgh and the surrounding areas either through the Community Health Index - a list of individuals registered with a General Practitioner - or as volunteers replying to media calls. Ethics Committee. The first wave of follow-up ran from 1999-2001, and 550 individuals (234 men and 316 women) were tested individually at the Wellcome Trust Clinical Research Facility (WTCRF) at the Western General Hospital, Edinburgh. The mean age of the LBC1921 participants was 10.9 years (s.d. 0.3) at the time of the SMS 1932 and 79.1 years (s.d. 0.6) at wave 1 assessment.

In the SMS1932, all participants completed the Moray House Test (MHT) Number 12 which was re-administered at age 79. The Moray House Test is a well-validated IQtype test with a predominance of verbal reasoning items, though there are also some other types of item. The raw MHT scores were corrected for age in days at the time of testing and then converted into IQ scores. At wave 1, a further battery of cognitive tests was also completed, including the Raven's Progressive Matrices to assess nonverbal reasoning, Verbal Fluency to assess executive function, and Logical Memory to assess verbal declarative memory. The Mini-Mental State Examination as a brief screen for dementia and the Hospital Anxiety and Depression Scale were also administered.

In the present study, only the association with IQ score, logical memory (immediate, delayed and total), verbal fluency and Raven's matrices were analyzed as these variables were the most comparable to the variables analyzed in the NCNG sample.

**The Lothian Birth Cohort 1936 (LBC1936)** comprises 1091 participants who were born in 1936 and tested on a general measure of verbal reasoning (Moray House Test No. 12; MHT) at age 11 in the Scottish Mental Survey of 1947 (Scottish Council for Research in Education [SCRE]. They were recruited in Edinburgh and the surrounding areas either through the Community Health Index -a list of individuals registered with a General Practitioner- or as volunteers replying to media calls. All participants lived independently in the community and were able to travel to the clinical research facility for testing. They undertook medical and cognitive testing at age 70 (mean age of 69.6 years, s.d. 0.8) including the same MHT test as they had taken at age 11. The raw MHT scores were corrected for age in days at the time of testing and then converted into IQ scores. The Mini-Mental State Examination (MMSE) was used to screen for possible dementia. The battery of cognitive tests sampled a variety of specific cognitive abilities, with an emphasis on memory and processing speed. Memory domains were assessed by the following subtests of the Wechsler Memory Scale-IIIUK (WMS-IIIUK: Logical Memory I (immediate verbal declarative memory), Logical Memory II (delayed verbal declarative memory), Verbal paired associates (immediate and delayed verbal memory and learning) and Spatial span (non-verbal, spatial memory). The information processing speed battery comprised two psychometric tests from the WAIS-IIIUK (Digit symbol coding and Symbol search) and two elementary cognitive tasks, Reaction Time (simple and choice conditions) and Inspection Time. Other cognitive tests which tapped diverse abilities included: Backward digit span (working memory) from the Wechsler Memory Scale-IIIUK; and Letter-number sequencing (working memory), Matrix reasoning (non-verbal reasoning) and Block design (constructional ability) from the WAIS-IIIUK. The Verbal fluency test provided a measure of executive function.The g factor was calculated via principal components analysis of the following Wechsler tests: Backward digit span, Letter-number sequencing, Matrix reasoning, Block design, Digit Symbol and Symbol Search subtests. The final sample for analysis (i.e., those who also had genotype data) was N = 1077, and included 535 females and 542 males.

### 2. Selection, genotyping and analysis of genetic markers.

**Markers selection, genotyping and analysis in the NCNG sample.** Haplotype tagging markers (single nucleotide polymorphisms - SNP) were selected using the Phase I (16c.1, June05, based on NCBI B34 assembly, dbSNP b124) version of HapMap (http://www.hapmap.org/cgi-perl/gbrowse/gbrowse/hapmap_phaseI/) .(The International HapMap Consortium. Nature 2005; 437: 1299-1320.), and according to the protocol described in Christoforou et al. (Christoforou A, *Mol Psychiatry* 2007 Nov; **12**(11): 1011-1025). The markers were genotyped on a Sequenom Massarray platformTM (http://www.sequenom.com/, Sequenom Inc., San Diego, CA, USA) at CIGENE, Center for Integrative Genetics (Universitetet for miljø- og biovitenskap, As, Norway, http://www.umb.no/), which is the national FUGE platform for genotyping (www.fuge.no), supported by the Research Council of Norway.

Genotypes were quality controlled with the following criteria: individual samples with genotype call rate < 90% and markers with call rate < 96 % were excluded from analysis.

### Markers selection, genotyping and analysis in the LBC1921 and LBC1936 samples.

Nine markers that showed association in the NCNG sample (p-value < 0.01: rs4591003, rs1926467, rs2296645, rs943220, rs10507435, rs7989245, rs7323560, rs7334245, rs9315383) were selected for replication in the LBC samples. In addition, we chose to include another seven markers for genotyping in these samples based on their relevance for ongoing studies in our lab (rs12430800, rs4391923, rs10492555, rs872060, rs9545332, rs7989807, rs9315390).
Assays were developed for the ABI PRISM® 7900HT Sequence Detection System using TaqMan technology (Applied Biosystems, CA, USA), with genotyping at the Welcome Trust Clinical Research Facility, Genetics Core (http://www.wtcrf.ed.ac.uk/genetics/default%20genetics.htm, University of Edinburgh, UK).

### Data analysis

Genotyping data were analyzed using the Helix Tree software for linear regression, genotypic association and haplotype trend regression of 2- and 3- markers sliding windows. Markers with Hardy-Weinberg equilibrium p-value < 0.001 were excluded.

### 3. RT-PCR of human DCLK1 transcripts.

cDNA samples were synthesized from human brain region-specific total RNA samples (Clontech Laboratories, CA, USA) (200 ng RNA input) using the SuperScript III First-Strand Synthesis System (Life Technologies, St Paul, MN, USA), according to manufacturer's protocol (20 µl reaction volume). 0,5 µl cDNA was used as template in 10 µl real-time PCR assays (50°C/2 min; 95°C/10 min; 40x(95°C/15 sec; 60°C/1 min)) . Real-time PCR analyses were performed with an ABI Prism 7900HT sequence detector system (Applied Biosystems, CA, USA) using SYBR-green (Eurogentec, Belgium) as detector, AmpliTaq Gold DNA polymerase (Applied Biosystems, CA, USA), 2x SYBR-green mix, and the following PCR primer sets:
*CARP*: 5'-GGATGACTTGGATTCAGTAGGAGACT (Seq. ID No. 3),
5'-CATGGTTAGTGTGTTCTTGTACTCAATATT (Seq. ID No. 4);
long *DCLK1*: 5'-GGAGTGGTGAAACGCCTGTAC (Seq. ID No. 5),
5'-GGTTCCATTAACTGAGCTGG (Seq. ID No. 6);
short *DCLK1*: 5'-ACACTAAGACTGTGTCCATGTTAGAACTC (Seq. ID No. 7),
5'-AAGCCTTCCTCCGACACTTCT (Seq. ID No. 8).

The specificity of all RT-PCR assays was verified by DNA-sequencing of the amplified PCR products.

### 4. Semi-quantitative RT-PCR of BDNF-induced expression of Dclk1 in vivo.

The expression of *Dclkl* transcripts in the rat hippocampus were measured by semiquantitative RT-PCR on cDNA samples obtained from our previous study on BDNFmediated induction of Long-Term Potentiation in the rat hippocampus. PCR were performed as described above with the following primer sets:
*Dclkl* long: 5'-GGTGTGGTGAAGCGTCTGTAC (Seq. ID No. 5), 5'-CAAAAAAGTCCTGAAGGCACATC (Seq. ID No. 6);
*Dclkl* short: 5'-ACACTAAGACTGTGTCCATGTTAGAACTC (Seq. ID No. 7), 5-GGCCATCGTTCTCATCCATT (Seq. ID No. 8).

### 5. Sequencing of intron 5 of human DCLK1.

Genomic DNAs of 23 individuals selected for their genotypes at the markers rs943220 and rs10507435 from the NCNG sample were amplified to sequence the DCLK1 intron 5. Nineteen pairs of primers for PCR amplification of the intron 5 were designed, using the Primer3 tool (http://fokker.wi.mit.edu/primer3/input.htm). The PCR amplicons were designed to have a sufficient (100-150 bp) overlap to obtain contiguous sequences. PCR fragments were amplified using AmpliTaq Gold (Applied Biosystems, Foster City, CA, USA) according to the manufacturer's instructions with 25 cycles: 94°C for 10 s, 55°C for 30 s, 72°C for 30 s (initial denaturation 94°C for 10 min). The PCR products were sequenced with BigDye v3.1 (Applied Biosystems) according to the manufacturer's instructions and the sequences were aligned with phredPhrap program and read in Consed39 (http://bozeman.mbt.washington.edu/consed/consed.html#documentation).

### 6. In silico prediction of promoter and cis-regulatory regions in intron 5 of human DCLK1.

Candidate gene-regulatory regions were identified on the basis of clustering of transcription factor (TF) DNA-binding sites, as identified by the TF-search engine (www.cbrc.jp/research/db/TFSEARCH) and the Cis-element Cluster Finder (Cister)- program (http://zlab.bu.edu/%7Emfrith/cister.shtml) using matrixes from TRANSFAC (http://www.gene-regulation.com). TFs predicted to bind proximal to SNPs verified by sequencing in the human *DCLK1* intron 5 were selected for the Cister-analyses. The Neuronal Network Promoter Prediction program was used to rank candidate TATA-boxes in intron 5 (http://www.fruitfly.org/seq_tools/promoter.html). Probability scores of clustering between promoter elements and TATA-boxes were investigated by the Cister-program and the TF-search engine.

### 7. Construction, transfections and assays of the Luciferase reporter vectors.

### Construction of luciferase reporter vectors.

The predicted promoter fragments were amplified from genomic DNA (from 2 individuals homozygous for the C or T allele of rs4391923/marker m5.3) by PCR with AmpliTaq Gold DNA polymerase (Applied Biosystems), using the forward primer
5'-CTAGACTCGAGCCTCCTGAAGATAGCTTTGC (Seq. ID No. 9) and the reverse primer 5'- CAGACAAGCTTCAGTCTCAGGAATACCTTGC (Seq. ID No. 10) (XhoI and HindIII sites introduced).

Purified PCR fragments were cloned into the XhoI/HindIII (all restriction enzymes from New England Biolabs, Ipswich, MA, USA) sites of 14 pGL4.11[luc2P] (Promega, Madison, WI, USA), generating the pGL4.11-Cprom and pGL4.11-Tprom luciferase reporters. Three haplotypes of the intron 5 cis2 element (hap1a, hap1b and hap2, see section 6 and supplementary online material - SOM) were PCR amplified from genomic DNA (from 3 individuals homozygous for the three haplotypes identified by sequencing), using the forward primer 5'-GTCACGGATCCTTGGAAACTCAAGAAGATAGGC (Seq. ID No. 11) and the reverse primer 5'-GATCAGTCGACCCACAGGAAACAAAGCAACC (Seq. ID No. 12) (BamHI and Sal1 sites introduced). Amplified DNA was cloned into the BamH1/Sal1 sites of pGL4.11-Cprom, pGL4.11-Tprom and pGL4.11[1uc2P], generating haplotype specific cis2-C/Tprom luciferase reporters and promoter-less control vectors. In the final plasmids, the Cis2 element locates 2216 bp upstream of the promoter-region, the two being separated by a synthetic poly(A) sequence for the reduction of background signals. All plasmid constructions were verified by DNA sequencing.

### Cell cultures, differentiation and plasmid transfection.

SH-SY5Y cells (ATCC, LGC Promochem, UK) were grown in RPMI supplemented with 10% (v/v) horse fetal serum, 5% (v/v) fetal bovine serum and 2 mM L-glutamine (Cambrex Biosciences, Cambrex Corporation, Charles City, IA, USA). Cultures were incubated at 37°C in a 5 vol-% CO2/air incubator. Plasmid transfections were performed over-night using MetafecteneTM Pro (Biontex,Munchen, Germany) under conditions optimized according to the manufacturer's guidelines. Neuronal differentiation was initiated the day after transfection by replacing the transfection media with fresh media containing BDNF (50 ng/ml)/RA (10µM all-trans retinoic acid) (Sigma-Aldrich, St Louis, MO, USA). Control cells (nondifferentiated) were treated similarly but exposed to media with vehicle only (0.01 % DMSO). Cell viability was monitored using WST-1 (F. Hoffmann-La Roche Ltd, Germany). All transfections were performed in quadruplicates on 96 multi-well plates. Each experiment was controlled for transfection efficiency by transfecting a green fluorescent protein (GFP) expressing vector (pSIREN-RetroQ-ZsGreen) into a separate set of cells. GFP expression was analyzed two days post transfection on a FACSCalibur™ Flow Cytometer (BD systems, BD Europe, Belgium).

### Luciferase Reporter Assays.

The transcriptional activity of each reporter plasmid was measured two days post transfection using the Dual-Luciferase Reporter Assay system (Promega Biotech AB, Madison, WI, USA), according to manufacturer's protocol. Luciferase signal intensities were recorded on a Chameleon plate reader (Reactionlab Sverige AB, Sweden). All plasmid transfections and luciferase measurements were performed in 96 multiwell plates, with quadruplicates of each sample. Each experiment was repeated 5 times for the pGL4-C/Tprom plasmids, and 7 times for plasmids harboring cis2 elements. All experiments were performed blind to the vectors haplotypes. Luciferase signal intensities were normalized for transfection efficiency on the basis of renilla luciferase signal intensities from the cotransfected pGL4-73[hRluc/SV40] plasmid. The transcriptional activity of a given reporter plasmid is listed relative to its respective promoter-less control vector. All cis2 promoter-less control vectors showed similar background activity.

Functional convergent genomics approaches, which use a set of candidate genes identified in gene expression-based, relevant models (Le-Niculescu H, Neurosc Biobehav R 2007; 31: 897-903), have been successful to identify new genes for schizophrenia, antipsychotic induced weight gain and bipolar disorders (Le-Niculescu Am J Med Genet 2009; 150B (2): 155-181). In order to broaden the study of *BDNF* in cognitive traits, we chose to develop a functional convergent genomics approach to characterise genes upregulated by BDNF, and their implication in cognition. BDNF plays a critical role as a trigger of memory formation and transcription-dependent enhancement of synaptic strength, via neuronal activity induced gene expression. Previously, we used a rat model where BDNF is infused in the dentate gyrus region of the hippocampal formation, and identified a panel of genes strongly up-regulated following BDNF treatment that induces long-term potentiation - LTP (Wibrand K, et al., Eur J Neurosci 2006 Mar; 23(6): 1501-1511). Here, haplotype-tagging SNPs to screen those genes (*ARC, BDNF, NEURITIN, DCLK1, KLF10* and *NPTX2)* for influence on human cognitive functioning in samples of healthy individuals who volunteered for testing of their memory and general intellectual function (IQ) has been selected.

### RESULTS

### Association to verbal memory and general cognition in the NCNG sample.

A total of 46 markers were genotyped in the Norwegian Cognitive NeuroGenetics - NCNG -sample. This sample consists of 271 individuals (mean age: 62.6 years, range 50-75), which had been recruited via media advertisements and who were subjected to cognitive testing, i.e. verbal memory and general cognition (IQ score). In this sample, we found strong associations between several markers in the *DCLK1* gene (doublecortin- and calmodulin kinase like 1, a.k.a. *DCAMKL1*) and aspects of verbal memory function and IQ score, which resisted Bonferroni correction (see Table 1, Table 2 for results of markers tested). The most significant associations were observed for intron 5 markers m5.1 and m5.2, where the less frequent genotypes were significantly associated with reduced verbal memory performance (see Table 1). Marker m5.1 was also associated with IQ score, like several other *DCLK1* SNPs (see Table 2). For the other genes tested we found some significant effects, but no strong association (p< 0.01) or associations across several cognitive traits. However, in a 2-loci analysis for gene-gene interaction effects, there were significant interactions between *DCLK1* intron 5 markers (m5.1 and m5.2) and markers tagging *BDNF* and *ARC* on the association with verbal memory (p-value = 0.03 - 0.003, after Bonferroni correction, see SOM). At the genetic level, this finding is consistent with the co-upregulation of *Dclkl* transcription by Arc and BDNF, as observed in a rat hippocampal model of synapse consolidation.

### Replication in the Lothian Birth Cohorts.

Sixteen *DCLK1* SNPs were selected for replication testing in two independent samples. The Scottish Lothian Birth Cohort (LBC) studies of individuals born in 1921 and in 1936 (LBC1921 and LBC1936) are two independent cohorts of individuals who underwent an IQ test at age 11 (IQ11), and who at the age of 79 years for the LBC1921, and 70 years for the LBC1936, participated in follow-up testing with examination of IQ (IQ79 and IQ70, respectively) and other cognitive functions.

In the LBC1921, we found an association between verbal memory and m11, an exon 11 synonymous marker (see Table 1). For IQ79, especially when regressed for IQ11, we replicated and further strengthened the association to markers in the intron 15 and intron 19 (which are located in the same haplotype block), and to intron 19 3-markers haplotype (resisting permutation testing, see Table 2).

In the LBC1936, we observed associations between an intron 5 marker (m5.3) and several memory-specific and general cognition traits at age 70 (see Table 1, and 2). Also in this sample, the association with intron 5 was even stronger (resisting permutation testing), for both memory and general cognition traits, at the haplotype level (3-markers haplotype covering the intron 5, see Table 2). In this sample, the intron 15 - intron 19 markers (except for m19.1) were associated with childhood cognitive variables (IQ11). Further association testing with other cognitive abilities (see SOM) showed an effect of markers in both the intron 5 and intron 15-19, but also an effect of the age at testing. This age dependent association is especially pronounced for the general cognitive ability factor, g factor, which was associated with both intron 5 (m5.3) and intron 15 - intron 19, but only 18 the intron 5 association remained significant when IQ11 was adjusted for in the regression model.

### Expression of rat Dclk1 variants after BDNF treatment in the dentate gyrus.

The rodent *Dclkl* gene is expressed as several transcripts, e.g., *long* (exons 1-20 except 6 and *8), short* (exons 6-20 except 8) and *Carp* (exons 6-8; see figure 1A), which vary in expression (spatial and developmental) and in function (see below).
In rat, it was previously demonstrated that the *Carp* variant is induced by BDNF exposure, but at this time we did not further look at the other transcripts. Further examination of the samples from that study, show that all short *DCLK1* variants are induced by BDNF but not the long variants (see figure 1B).

### Expression of DCLK1 variants in human brain tissues.

In the rodent, the short variants show higher expression in adult brains whereas long variants dominate during embryonic stages in rat (Burgess HA, Reiner O. J Biol Chem 2002 May; 277(20): 17696-17705). In humans, the expression of *DCLK1* variants has not been fully documented, but expression of long *DCLK1* has been shown in both embryonic and adult brain tissues. Using transcript-specific PCR assays, we found expression of both long and short transcripts of *DCLK1* in the fetal brain (26-40 weeks) as well as in specific regions of the adult human brain (see Figure 1 C). The expression of short variants was especially high in regions involved in memory performance (hippocampus, occipital pole, frontal lobe and temporal lobe). The expression of *CARP* was low in all human brain tissues, consistent with previous observations in rodents of low basal *CARP* expression and robust induction by specific treatments.

### In silico characterisation of regulatory elements and identification of additional genetic variants in the intron 5 of DCLK1.

Considering that one of the main signals of association was observed for markers in the intron 5 of *DCLK1* and that short transcripts of *DCLK1* all start from the exon 6, we hypothesised that the intron 5 might harbour alternative promoter and regulatory regions. We searched for transcriptional cis-regulatory elements and promoters in the human intron 5 with the aim of analyzing the effect of these in luciferase reporter assays. The search predicted a TATA-box promoter and three specific cis-regulatory elements (cis1-3, see Figure 2A). To identify additional sequence variants that could affect the regulatory elements, we sequenced the entire intron 5 from genomic DNA of 23 individuals from the NCNG sample. In the putative alternative promoter region in intron 5, the marker m5.3 (C/T variant) was located 2 bp from the potential transcription start. For the adjacent regulatory elements we observed several variants in the cis2 that could affect the regulatory properties, defining three haplotypes: the related hap1a and hap1b, and the hap2.

### Assessment of the functional effect of SNPs in potential regulatory regions on the expression of DCLK1 short variants.

Luciferase reporter assays were constructed by cloning the promoter C (C-prom) or T allele (T-prom) of m5.3, from genomic DNAs of NCNG individuals. Additional reporter assays were constructed to mimic *in vitro* the potential effect of the 3 cis2 haplotypes on the promoter. The assays were analysed for expression of the reporter luciferase protein under control conditions and under BDNF/RA differentiation (retinoic acid, protocol for BDNF induced differentiation as described by Holback et al. (Holback S, er al., J Neurochem 2005 Nov; 95(4): 1059-1068).

In luciferase reporter assays, both the C-prom and T-prom constructs displayed basic promoter reporter-activity in the undifferentiated SH-SY5Y control cells. However, during BDNF-induced neuronal differentiation, only the T-prom construct demonstrated increased expression of the luciferase reporter (see Figure 2C). The addition of a single hap1a or hap1b variant of the cis2 element into the non-inducible C-prom reporter-vector rendered the C-prom inducible by BDNF (see Figure 2D), whereas the hap2 variant of cis2 did not show this effect on C-prom. None of the cis2 variants had any significant additional effect on the activity and inducibility of the Tpromequence. These reporter assays thus demonstrated that the predicted promoter in intron 5 displays an allele-specific promoter activity and inducibility which can be further influenced by regulatory elements, such as cis2, in an allele-specific mode.

Our data show that genetic variants in *DCLK1* significantly influence the performance on tests of memory and intellectual function in three independent samples. We observed some differences between the samples regarding the panel of markers that were associated, but although the samples have been phenotyped for similar traits (e.g. verbal memory and general cognition), it is likely that differences in the origin, age, or mode of recruitment, as well as differences in the specific tests used to assess cognitive phenotypes, may in part explain these differences.

The thorough examination of the Lothian Birth Cohorts, especially the LBC1936, shows that the signal of association depends on the cognitive variable studied and on the age at examination, which points to similarities and differences in genetic contributions to cognitive abilities across the lifespan. Several cognitive associations in old age were affected by the integration of childhood IQ as a covariate, which can suggest that these are genetic associations to lifelong cognitive change.

In addition, we show that there might be interaction between variants in *DCLK1* and variants in *BDNF* and *ARC* for an effect on verbal memory and general cognition. This finding is coherent at the genetic level with the *in vivo* observation that *Dclkl* is co-upregulated with *Arc* by BDNF in the rat hippocampus. In addition to the known implication of BDNF in long term potentiation, the interactions with Arc are also noteworthy as this immediate early gene is required for multiple forms of synaptic plasticity and long-term memory formation, including synaptic potentiation induced by BDNF infusion.

The rodent *Dclk1* gene is expressed as several transcripts, e.g., *long* (exons 1-20 except 6 and 8), *short* (exons 6-20 except 8) and *Carp* (exons 6-8; see figure 1A), which vary in expression (spatial and developmental) and in function. The long transcripts encode an N-terminal domain similar to the lissencephalia-related doublecortin gene, sharing its microtubule-binding and -stabilizing properties. N-terminal *Dclk1* transgenic and *Dcl* knockdown mice develop brain abnormalities that affect the organization of hippocampal neurons, cortical neurogenesis, neuronal migration and axonal wiring. The C-terminal domain, present in both long- and short-*Dclk1*, contains a domain similar to the Ca2+/calmodulin-dependent protein kinase which may phosphorylate the myelin basic protein. The function of Carp remains largely unknown but it is expressed in response to diverse stimuli, and may be involved in both neuronal activity-induced strengthening of synaptic transmission as well as apoptosis. We now show that the short variants are, similarly to *Carp,* up-regulated by BDNF treatment in the rat hippocampus, and that in human these variants are expressed in brain structures relevant to cognition. We also characterise an alternative promoter in one of the associated region, intron 5, which is used for transcription of the short variants. The efficiency of these regulatory elements to express the reporter gene was influenced by the alleles of the associated markers and by the treatment with BDNF.

In this context, it is interesting to note that the BDNF- inducible C-prom/cis2 haplotypes (hap 1a/b), equivalent to 2-marker m5.1-m5.3_GA haplotype of intron 5 shows the strongest association to IQ score and verbal memory (p-value = 0.0027 and 0.0019 in the LBC1936), and that the "non-inducible" GG haplotype is the rarest, found only in 2.5% of the chromosomes.

Herein, we also observed association of markers in a linkage disequilibrium block covering intron 15 to intron 19 to variation in cognitive abilities. It is interesting to note that the associated markers are located near exon 19 that is alternatively spliced in several transcripts, affecting the kinase activity within a region of high inter-species conservation (as seen in the UCSC: http://genome.ucsc.edu/cgi-bin/hgGateway).

**Table 1. Genotypic single marker association analysis to verbal traits**

| | | | | | NCNG sample (N = 271) | | LBC1921 (n = 550) | LBC1936 (N = 1077) |
|---|---|---|---|---|---|---|---|---|
| | Markers | | | | CVLTII Learning | CVLTII Delayed recall | LM Delayed | LM Learning |
| Gene | tested | associated | code | localization | | | | |
| *NRNI* | 7 | - | - | - | - | - | n. t. | n. t. |
| *NPTX2* | 3 | - | - | - | - | - | n. t. | n. t. |
| *KLF10* | 4 | - | - | - | - | - | n. t. | n. t. |
| *ARC* | 2 | - | - | - | - | - | n. t. | n. t. |
| *BDNF* | 6 | - | - | - | - | - | n. t. | n. t. |
| *DCLK1* | 28 | rs9315383 | m3.1 | intron 3 | 0.0033 | - | - | - |
| | | rs7334245 | m3.2 | intron 3 | 0.0059 | - | - | - |
| | | rs7989245 | m4 | intron 4 | - | - | - | - |
| | | rs10507435 | m5.1 | intron 5 | 0.0021 | 0.00043 | - | - |
| | | rs943220 | m5.2 | intron 5 | 0.0036 | 0.00010 | - | - |
| | | rs4391923 | m5.3 | intron 5 | - | - | - | 0.0067 |
| | | rs2296645 | m11 | exon 11 | | | 0.0068 | |

Analyses were performed with Helix Tree software. Only p-values <0.01 are reported. In the NCNG sample, verbal learning and delayed recall were assessed with the California Verbal Learning Test (CVLT-II). In total, six BDNF-LTP related genes were tested and 48 markers were analyzed. In the LBC samples, verbal memory was tested with the Wechsler memory scale test (LM - delayed and learning) and the samples were genotyped for 16 DCLK1 markers only, with no screening of the other genes (n. t.: not tested). P-values below Bonferroni corrected p-value threshold (=0.001 for NCNG), are highlighted in bold.

**Table 2. Association of DCLK1 markers to IQ scores in the three samples.**

| | | | | | | | | | | | | | |
|---|---|---|---|---|---|---|---|---|---|---|---|---|---|
| | | | | NCNG | | LBC1921 | | | | LBC1936 | | | |
| | | | | IQ | | IQ79 | | IQ79 rIQ11 | | IQ11 | | IQ70 | |
| | Markers | Code | Localisation | LR | HTR3 | LR | HTR3 | LR | HTR3 | LR | HRT3 | LR | H |
| | rs10492555 | m5'.1 | 5' | - | 0.034 | - | - | - | - | - | - | - | - |
| | rs9315390 | m5'.2 | 5' | - | 0.010 | - | - | - | - | - | - | - | - |
| | rs9315383 | m3.1 | Intron 3 | 0.005 | - | - | - | - | - | - | - | - | - |
| | rs7334245 | m3.2 | Intron 3 | - | - | - | - | - | - | - | - | - | - |
| | rs7989807 | m3.3 | Intron 3 | - | - | - | - | - | - | - | - | - | - |
| | rs7323560 | m3.4 | Intron 3 | - | - | - | - | - | - | - | - | - | - |
| | rs7989245 | m4 | Intron 4 | - | - | - | - | - | - | - | - | - | - |
| | rs10507435 | m5.1 | Intron 5 | 0.027 | - | - | - | - | - | - | - | - | 0. |
| | rs943220 | m5.2 | Intron 5 | - | - | - | - | - | - | - | - | - | - |
| | rs4391923 | m5.3 | Intron 5 | - | - | - | - | - | - | - | - | 0.010 | - |
| | rs2296645 | m11 | Exon 11 | - | 0.046 | - | - | - | - | - | 0.045 | - | - |
| | rs1926467 | m15 | Intron 15 | 0.625 | - | - | - | 0.014 | - | 0.023 | - | - | - |
| | rs12430800 | m19.1 | Intron 19 | - | 0.019 | 0.0042 | 0.022 | 0.0019 | 0.00034 | - | - | - | - |
| | rs4591003 | m19.2 | Intron 19 | - | - | - | 0.014 | 0.039 | - | 0.015 | - | - | - |
| | rs9545332 | m19.3 | Intron 19 | - | - | - | - | - | - | 0.023 | - | - | - |
| | rs872060 | m3' | 3' | - | - | 0.026 | - | 0.038 | - | - | - | - | - |

Both single marker linear regression (LR) and haplotype trend regression of 3-markers sliding windows analyses (HTR3) are presented. Only p-values below 0.05 are displayed. All analyses were performed using sex and age as covariates. The NCNG was assessed with the Wechsler Abbreviated Scale of Intelligence. LBC1921 and LBC1936 were assessed for IQ with the Moray House Test. For LBC1921, no association below p=0.05 was observed for IQ11. In the LBC samples, all regression analyses were performed both with and without IQ11 as a covariate. P-values highlighted in bold are resisting a 10,000 permutations testing.

## Claims

1. A use of SNPs present in the DCLK1 gene according to Seq. ID No. 1 for determining cognitive abilities and/or cognitive performance in an individual.

2. The use according to claim 1 wherein the SNP is at least one SNP as shown in table 1 or table 2.

3. The use according to claim 1 or 2 wherein the cognitive abilities and / or cognitive performance are selected from memory, learning, schizophrenia, hyperactivity, bipolar affective disorder, in particular, memory or learning.

4. A method for testing and / or determining the cognitive abilities and / or cognitive performance of an individual comprising the step of identifying the presence or absence of SNPs in the DCLK1 gene according to Seq. ID No. 1 in an individual, and determining the cognitive abilities and/or cognitive performance based on the presence or absence of a SNP.

5. A method according to claim 4 wherein the SNP is at least one of the SNP shown in Table 1 or table 2.

6. A method according to claim 4 or 5 wherein the cognitive abilities and / or cognitive performance is at least one of memory, learning, schizophrenia, hyperactivity, bipolar affective disorder, in particular, memory or learning.

7. System for determining the cognitive ability and / or cognitive performance of an individual comprising means for identifying SNPs in the DCLK1 gene of Seq. ID No. 1 and instructions to assign a SNP present in the DCLK1 gene with the cognitive ability and/or cognitive performance.

8. The system according to claim 7 wherein the SNP to be identified are at least one of the SNPs shown in table 1 or table 2.

9. A use of compounds able to increase the transcription or expression of the DCLK1 gene or DCLK1 gene product for improving cognitive abilities and/or cognitive performance in an individual.

10. The use according to claim 9 wherein the cognitive abilities and/or cognitive performance are selected from memory, learning, schizophrenia, hyperactivity and bipolar affective disorder.

11. The use according to claim 9 or 10 wherein said compound is BDNF or compounds increasing BDNF expression.

12. A method for screening compounds for preventing or treating cognitive deficits or cognitve decline, in parituclar, age-based cognitive decline comprising the step of determining the ability of candidate compounds to alter expression and/or activity of DCLK1.

13. A method for identifying compounds useful as pharmaceuticals for the prophylaxis or treatment of cognitive deficits and/or cognitive decline comprising the step of determining the ability of a candidate compound to alter the expression or acitivity of DCLK1.

14. The method of claim 12 or 13 wherein the candidate compound has the ability to increase DCLK1 expression and/or acitivity.

15. The method according to any one of claims 12 to 14 wherein the compound is selected from RNA, DNA or small molecules.


SEQUENCE LISTING

<110> Bergen Teknologioverforing AS

<120> DCLK1 and cognitive ability and cognitive performance

<130> 4200-007 EP-1

<160> 28

<170> PatentIn version 3.3

<210> 1
<211> 8082
<212> DNA
<213> human

<220>
<221> CDS
<222> (234)..(2423)

<400> 1
tttcaatgag gacgggccga ggcacatccc tgcactagtg gccgcaaccg aggcgccgcg 60

ctccagcagc tgctgccgcc cagcccggcc ccgccgccgc cccccagccc tgcagccccg 120

cagccccggc cgcgcccagc ccggcgagga cagcaccagg aggcggcccc cagcgcggcc 180

acaaagaccc ccggcggcgt ctctccgcgg accggtccta cttgaagtcc atc atg 236
Met
1

tcc ttc ggc aga gac atg gag ctg gag cac ttc gac gag cgg gat aag 284
Ser Phe Gly Arg Asp Met Glu Leu Glu His Phe Asp Glu Arg Asp Lys
            5                   10                  15

gcg cag aga tac agc cga ggg tcg cgg gtg aac ggc ctg ccg agc ccg 332
Ala Gln Arg Tyr Ser Arg Gly Ser Arg Val Asn Gly Leu Pro Ser Pro
        20                  25                  30

acg cac agc gcc cac tgc agc ttc tac cgc acc cgc acg ctg cag acg 380
Thr His Ser Ala His Cys Ser Phe Tyr Arg Thr Arg Thr Leu Gln Thr
    35                  40                  45

ctc agc tcc gag aag aag gcc aag aaa gtt cgt ttc tat cga aac gga 428
Leu Ser Ser Glu Lys Lys Ala Lys Lys Val Arg Phe Tyr Arg Asn Gly
50                  55                  60                  65

gat cga tac ttc aaa ggg att gtg tat gcc atc tcc cca gac cgg ttc 476
Asp Arg Tyr Phe Lys Gly Ile Val Tyr Ala Ile Ser Pro Asp Arg Phe
                70                  75                  80

cga tct ttt gag gcc ctg ctg gct gat ttg acc cga act ctg tcg gat 524
Arg Ser Phe Glu Ala Leu Leu Ala Asp Leu Thr Arg Thr Leu Ser Asp
            85                  90                  95

aac gtg aat ttg ccc cag gga gtg aga aca atc tac acc att gat ggg 572
Asn Val Asn Leu Pro Gln Gly Val Arg Thr Ile Tyr Thr Ile Asp Gly
        100                 105                 110

ctc aag aag att tcc agc ctg gac caa ctg gtg gaa gga gag agt tat 620
Leu Lys Lys Ile Ser Ser Leu Asp Gln Leu Val Glu Gly Glu Ser Tyr
    115                 120                 125

gta tgt ggc tcc ata gag ccc ttc aag aaa ctg gag tac acc aag aat 668
Val Cys Gly Ser Ile Glu Pro Phe Lys Lys Leu Glu Tyr Thr Lys Asn
130                 135                 140                 145

gtg aac ccc aac tgg tcg gtg aac gtc aag acc acc tcg gct tct cgg 716
Val Asn Pro Asn Trp Ser Val Asn Val Lys Thr Thr Ser Ala Ser Arg
                150                 155                 160

gca gtg tct tca ctg gcc act gcc aaa gga agc cct tca gag gtg cga 764
Ala Val Ser Ser Leu Ala Thr Ala Lys Gly Ser Pro Ser Glu Val Arg
            165                 170                 175

gag aat aag gat ttc att cgg ccc aag ctg gtc acc atc atc aga agt 812
Glu Asn Lys Asp Phe Ile Arg Pro Lys Leu Val Thr Ile Ile Arg Ser
        180                 185                 190

ggc gtg aag cca cgg aaa gct gtc agg att ctg ctg aac aag aaa acg 860
Gly Val Lys Pro Arg Lys Ala Val Arg Ile Leu Leu Asn Lys Lys Thr
    195                 200                 205

gct cat tcc ttt gag cag gtc ctc acc gat atc acc gat gcc atc aag 908
Ala His Ser Phe Glu Gln Val Leu Thr Asp Ile Thr Asp Ala Ile Lys
210                 215                 220                 225

ctg gac tcg gga gtg gtg aaa cgc ctg tac acg ttg gat ggg aaa cag 956
Leu Asp Ser Gly Val Val Lys Arg Leu Tyr Thr Leu Asp Gly Lys Gln
                230                 235                 240

gtg atg tgc ctt cag gac ttt ttt ggt gat gat gac att ttt att gca 1004
Val Met Cys Leu Gln Asp Phe Phe Gly Asp Asp Asp Ile Phe Ile Ala
            245                 250                 255

tgt gga ccg gag aag ttc cgt tac cag gat gat ttc ttg cta gat gaa 1052
Cys Gly Pro Glu Lys Phe Arg Tyr Gln Asp Asp Phe Leu Leu Asp Glu
        260                 265                 270

agt gaa tgt cga gtg gta aag tcc act tct tac acc aaa ata gct tca 1100
Ser Glu Cys Arg Val Val Lys Ser Thr Ser Tyr Thr Lys Ile Ala Ser
    275                 280                 285

tca tcc cgc agg agc acc acc aag agc cca gga ccg tcc agg cgt agc 1148
Ser Ser Arg Arg Ser Thr Thr Lys Ser Pro Gly Pro Ser Arg Arg Ser
290                 295                 300                 305

aag tcc cct gcc tcc acc agc tca gtt aat gga acc cct ggt agt cag 1196
Lys Ser Pro Ala Ser Thr Ser Ser Val Asn Gly Thr Pro Gly Ser Gln
                310                 315                 320

ctc tct act ccg cgc tca ggc aag tcg cca agc cca tca ccc acc agc 1244
Leu Ser Thr Pro Arg Ser Gly Lys Ser Pro Ser Pro Ser Pro Thr Ser
            325                 330                 335

cca gga agc ctg cgg aag cag agg agc tct cag cat ggc ggc tcc tct 1292
Pro Gly Ser Leu Arg Lys Gln Arg Ser Ser Gln His Gly Gly Ser Ser
        340                 345                 350

acg tca ctt gcg tcc acc aaa gtc tgc agc tcg atg gat gag aac gat 1340
Thr Ser Leu Ala Ser Thr Lys Val Cys Ser Ser Met Asp Glu Asn Asp
    355                 360                 365

ggc cct gga gaa gaa gtg tcg gag gaa ggc ttc cag att cca gct aca 1388
Gly Pro Gly Glu Glu Val Ser Glu Glu Gly Phe Gln Ile Pro Ala Thr
370                 375                 380                 385

ata aca gaa cga tat aaa gtc gga aga aca ata gga gat gga aat ttt 1436
Ile Thr Glu Arg Tyr Lys Val Gly Arg Thr Ile Gly Asp Gly Asn Phe
                390                 395                 400

gct gtt gtc aag gaa tgt gta gaa aga tcg act gct aga gag tat gct 1484
Ala Val Val Lys Glu Cys Val Glu Arg Ser Thr Ala Arg Glu Tyr Ala
            405                 410                 415

ctg aaa att atc aag aaa agc aaa tgt cga ggc aaa gag cac atg atc 1532
Leu Lys Ile Ile Lys Lys Ser Lys Cys Arg Gly Lys Glu His Met Ile
        420                 425                 430

cag aat gaa gtg tct att tta aga aga gtg aag cat ccc aat atc gtt 1580
Gln Asn Glu Val Ser Ile Leu Arg Arg Val Lys His Pro Asn Ile Val
    435                 440                 445

ctt ctg att gag gag atg gat gtg cca act gaa ctg tat ctt gtc atg 1628
Leu Leu Ile Glu Glu Met Asp Val Pro Thr Glu Leu Tyr Leu Val Met
450                 455                 460                 465

gaa tta gta aag ggg gga gac ctt ttt gat gcc att act tcc act aac 1676
Glu Leu Val Lys Gly Gly Asp Leu Phe Asp Ala Ile Thr Ser Thr Asn
                470                 475                 480

aaa tac acc gag aga gac gcc agt ggg atg ctg tac aac cta gcc agc 1724
Lys Tyr Thr Glu Arg Asp Ala Ser Gly Met Leu Tyr Asn Leu Ala Ser
            485                 490                 495

gcc atc aaa tac ctg cat agc ctg aac atc gtc cac cgt gat atc aag 1772
Ala Ile Lys Tyr Leu His Ser Leu Asn Ile Val His Arg Asp Ile Lys
        500                 505                 510

cca gag aac ctg ctg gtg tat gag cac caa gat ggc agc aaa tca ctg 1820
Pro Glu Asn Leu Leu Val Tyr Glu His Gln Asp Gly Ser Lys Ser Leu
    515                 520                 525

aag ctg ggt gac ttt gga ctg gcc acc att gta gac ggc ccc ctg tac 1868
Lys Leu Gly Asp Phe Gly Leu Ala Thr Ile Val Asp Gly Pro Leu Tyr
530                 535                 540                 545

aca gtc tgt ggc acc cca aca tac gtg gct cca gaa atc att gca gag 1916
Thr Val Cys Gly Thr Pro Thr Tyr Val Ala Pro Glu Ile Ile Ala Glu
                550                 555                 560

act gga tac ggc ctc aag gtg gac atc tgg gca gca ggt gta atc act 1964
Thr Gly Tyr Gly Leu Lys Val Asp Ile Trp Ala Ala Gly Val Ile Thr
            565                 570                 575

tat atc ctg ctg tgt ggt ttc cct cca ttc cgt gga agt ggt gat gac 2012
Tyr Ile Leu Leu Cys Gly Phe Pro Pro Phe Arg Gly Ser Gly Asp Asp
        580                 585                 590

cag gag gtg ctt ttt gat cag att ttg atg ggg cag gtg gac ttt cct 2060
Gln Glu Val Leu Phe Asp Gln Ile Leu Met Gly Gln Val Asp Phe Pro
    595                 600                 605

tct cca tac tgg gat aat gtt tcc gat tct gca aag gag ctc att acc 2108
Ser Pro Tyr Trp Asp Asn Val Ser Asp Ser Ala Lys Glu Leu Ile Thr
610                 615                 620                 625

atg atg ctg ttg gtc gat gta gat cag cga ttt tct gct gtt caa gta 2156
Met Met Leu Leu Val Asp Val Asp Gln Arg Phe Ser Ala Val Gln Val
                630                 635                 640

ctt gag cat ccc tgg gtt aat gat gat ggc ctc cca gaa aat gaa cat 2204
Leu Glu His Pro Trp Val Asn Asp Asp Gly Leu Pro Glu Asn Glu His
            645                 650                 655

cag ctg tca gta gct gga aag ata aag aag cat ttc aac aca ggc ccc 2252
Gln Leu Ser Val Ala Gly Lys Ile Lys Lys His Phe Asn Thr Gly Pro
        660                 665                 670

aag ccg aat agc aca gca gct gga gtt tct gtc ata gca ctg gac cac 2300
Lys Pro Asn Ser Thr Ala Ala Gly Val Ser Val Ile Ala Leu Asp His
    675                 680                 685

ggg ttt acc atc aag aga tca ggg tct ttg gac tac tac cag caa cca     2348
Gly Phe Thr Ile Lys Arg Ser Gly Ser Leu Asp Tyr Tyr Gln Gln Pro
690                 695                 700                 705

gga atg tat tgg ata aga cca ccg ctc ttg ata agg aga ggc agg ttt     2396
Gly Met Tyr Trp Ile Arg Pro Pro Leu Leu Ile Arg Arg Gly Arg Phe
                710                 715                 720

tcc gac gaa gac gca acc agg atg tga ggagccggta caaggcgcag           2443
Ser Asp Glu Asp Ala Thr Arg Met
            725

ccagctcctc ccgaactcaa ctcggaatcg gaagactact ccccaagctc ctccgagact   2503

gttcgctccc ctaactcgcc cttttaataa gaccctttta ctcaaagtcc tagcttaacc    2563

ctttgagact ctgagatttt tttcccccaa atttgtgtaa aacagtttca tctgatctat   2623

ctagcgctca atgcttgaat ggcagaactg aaagtgtttt caggtatctt tgtagcggtt   2683

tccctttact gaataagatg acacgtggtg attgtgaaga tggtaatttg ctgctaatag   2743

agtcctcaaa gggttaaggc caatttgcaa ttttttttta aacttagaag caatgaatgt   2803

tttcatcagt caagctagga tctgcagtat gtaatatagc acttgttaac cctctgagtg   2863

catagaattt tattgagaat tcttgtttgg gaatttttca ggcctttgga tgtatacaca   2923

catgtttctt gattttactg cagatcaagg ggtgttgtta gatgctgaaa tgtccagaaa   2983

agaaggacat ttagaatgat atcttgtttg tccttttctg tgggtttaga acgtggcagg   3043

tttataactt cgacacacgc acggttcttt cttcttcaca atcctattca gaaacagatt   3103

ttttttttca ttagagatat gactgtcagt tgcagtgagt tctgcatccc aagtggaggg   3163

aattgggttt gtggcaaaga gcttgaccca ggaaatagat ggtgcccccc aaattgtctc   3223

cacatgaaga tgtactgatg acgccccaga aatgctgctt ccatatcagc tgctgctagc   3283

gccagcgcag actctcaggg agtcaccaca gcttgtcttg tgcttggtga gtgagggtct   3343

ctctactcag tgtcagacat ctacaggaaa gaaacaactg gtggaaaaga gcaataaatt   3403

gcccggtgct ctgcagggct ggaatttcaa acagaaagag ggaataagat cctgtgattt   3463

ttctcacctg cttttccacg cactgtggtc atcactgtgc aatctacatc tagtatgaaa   3523

tccacacata ggagagctgg ggcacaaggg gactggaggc agttgctttg caagatggct   3583

gaggagaaag cacactggga acacaatcca gaatgttcta acaataagtt ttcagtgaat   3643

aaaccactgg caagacaatt ccatgtgcac ctttaggtta cctatatagt ctcctaggaa   3703

gatcaggatg aaagacctag atgatacccc tgaggataaa acctccatcc cctaaaatga   3763

ttttttttaa ataccactgt ctttagctgt ccaggaggtc agagtgtttt ttctgtcttt   3823

gggccaagtc ctgtctgaga cctgtatttt cactcttgtt accaaatcta tctccctagt   3883

gcagtgtctc caggcctgag tttcttctgg aacagattcc attttagaat ggggattcac   3943

aggttctgtg catcaccaca gtgctcagag aggattctcc tggggtgtct tagaggcagg   4003

tgcccaactc aaatgtattc ccaaggtttg ctgggctctg ggatccacga gacaaccaga   4063

gagggatatc tcatgaaatt tgcatctggt ggctgaacag tacctatgtt ctctgttttg    4123

aatatacttt aatacctgag agtcttaaaa tttgtgaaca acgtttctat agtcctttat 4183
tttcaaatgc acattgatct tcacttgctg catttttact cttcaaccct gaaactatgg 4243
tctacattaa tatggatttt taaatcacat gtcattactt ttgcaacacc atcaccaaaa 4303
ttttttgctc ttttacattt aggttcatct ctgtggtctg tgttgtcctg acatgtaaaa 4363
agcatatcgt ttattgaggt ttttttcccc cccttttaga gcatccggaa gtgataacac 4423
gcaaaatcac aaagtagcat aaatcagtaa attagttgag ttgtttttgg gggggaggtg 4483
ggggtagggg gcacagaaca ccagaaagag tgttggtgtg taggtagatt ccatattaat 4543
gaggaacact gaactagttg gaaattactg ctttctctag aaatataaag caaagcacta 4603
ttccaaggct atggagtagc tctacagcct ggcctcaact ctaaaagtgt gaagaatgca 4663
atgggcagag acctacctgc agtggactgt cattttcctt tctttctctg aattactgct 4723
ttttctgtgg gcattaacta tattgctaca gcatctagtg tactgagcct gcggtgcatg 4783
gctcaggcct tttcccatcg acgtctaggg ggactctgga ccgtgtgaag ctagggggtg 4843
tttctcagca cactgcagaa gggcagctca gaagaatgca gggcccattc agcatgggga 4903
tcccagcaca tcactgtaga atttgagtga tctatgctga ataaacagtg gaatgtgacc 4963
agtcaagtag aaatcttgag taatcagatg gaatgcaatc tttctaacat taagctacca 5023
agatcctgaa tgtcagagat gtactcagag ggttaacaga caagcacaag gcatgctgac 5083
tacattggtg tatccagatt gctttgcttt tagccagtgc tttctaattt ttttctcgac 5143
attcttggga tagttcaagt ttgaaataat taagtggtgg tgttctttaa ggaatttcta 5203
taaccaaatt gatcttattt ttgatttcac ttatcataga acaaatatgt atcattatgg 5263
cagtgtatct atgtaattat caatttaatc atcaccaccg gtgtttccat attttttccc 5323
aagtatttaa tatagctctc ttatggtggt ggcctggtga tggggaccgt ctttctttta 5383
ctgacacatg accaatcata tggtattttc aagggaattt taagattcat cttttcagtt 5443
tgatagtaga ctagttaagg aagaactctt tcattacttg catcgtgtaa atcatctctg 5503
tagacatgtg ttcatattaa tgaacacatt ttttctcaac attgtagcag aaatcatttt 5563
attcgtcatg atcaatgaat atgtgatttg ctccagatcg ttagaaggaa aagtaagatt 5623
tcagtcatca aaaatgtttt taccgtagcc ctcatctaac ttacacgtgg tgcatattaa 5683
aataagcaga gaaaaaaaaa tgtgaataaa ctactgaaaa cacttggtgt tttgtgttca 5743
atgagacctt cctgcaacct gctccccatg ggtggcagtt aacaggccca tcagatattg 5803
ttgaaagaaa gcaatatatc catgaatgaa ggctaaaatt gcaatccttt accctttgag 5863
gcatatttca gttgaaaaca aaaagaaaag aaaatttggc ttagagggtc acagagctcc 5923
catatgacca agtctcaagc acattaaatc atggttgttt actggccaag ggcgtccact 5983
agacaactct atcccttgcg ctgaagctca atcgtgctga gggagagctt tcttaatatt 6043
actgtgttgc tcttagccct tctctgggtt aggatctgtc agcatttcta tgataaactc 6103
ctattctcaa aggtttttaa tttgaccata aaaatgtgcc ccaggctgaa gtttgctata 6163

cagggctgta ccaaagagtg aaggtttact tccttctctt tccaacttct tccccattct 6223
ccaaggaaaa gaacaacaaa aaaatctggt atggtccctc cttaatagtg atttcagaat 6283
tttggaaagc accaagatcc aagatggtag ttttaatgta gttactcatt cgcacacatt 6343
ttttaaattt aatgggtcac ctggcatata ttgtagataa catatctttt ctataatttg 6403
taagtcaata atttttttaa ctgctacatg atattttttt ttgcccaaag attttaaaag 6463
acttgaagtt ggtcagttca aaactcagat ttttctacac attgtctgcc atgtccatta 6523
ggagtttggg gaaaatactc tcacacagac ccttactttg catgcagttt agagggtaag 6583
atacgtgctt cttttgggga taaagatttc cttacttaat tgtcaaattt catggagcca 6643
ttctagtctg ttggggaaaa tagtgattaa aagcacttcc aaaattaaca ttttttgaca 6703
attcagatat gaaaagaagc aggggaaaat aatacacttt actcttttct tgcttaaagg 6763
caaacaaatc aatgaaactt gaggacacac taaacatttg ataactgcaa atgtgcttta 6823
aaaattggtt caatggtgct tacacatgaa acggtaacaa atggggttcc taggacgtca 6883
gaaggaatct ttagtttgta tgtaattaca cactagagga ggaggtgctt ttaagccagt 6943
cttttatttt taatcatctc aaatatgcaa ccatacatgc agtaacatta agggtcgtaa 7003
actggtggga aacaggaact tcagtggaga ggcttaaatg cctctggtta gagtgggggt 7063
ttttgtttgt ttgtttattg ttgggtttca acactgagca tcatttctgt gatcaagttt 7123
ctaactggca tgtgttttga tcatgaggtt taccatatct tgcccataca gacaaatgag 7183
agatctagtt tcattttgtt ccctaaagaa agaacactct ctaaaattaa atcatacctg 7243
taaatttctt cagcatttgt ttctgttcaa tgaaattgag acccttaatg ttgctttaat 7303
gtaaaattga atattttgtc tgtgatatac tttaataatt taaagtaagt aatagttcta 7363
aagtcttcac tgttgctact aagagaaaat agaattttaa aggtgatgat aaagatgcta 7423
taatgtcagt tcactccagt ccaatcaaat gtagtaagaa aaagtccttg aatagttctc 7483
tagggacaat ttctcacttg ccattgacat taatctttgg tgtattctca gaaaaaataa 7543
aaagaaattg aaactggtcc aaggttatag tcatatcctc gataactttt gaaaaaaaat 7603
tttattagga aattaatact agcctttttc attctggctg aaagaaaatt attaaaggat 7663
tagttgagtg tgaaattcaa cagtattttg ctcatacata ctaaaaaggt gcgtagggac 7723
ttggcgcatt taaacaagtt tctgaaaggt ttcaatttga ctcaagaaaa aaattcaata 7783
tttcttttga aaatactgaa tttatcactt gctgcatgga tcagatggca taggttaatc 7843
tttgattttc agaatcctaa tgaaataact ttcaaacaat ttgtgtcctt aattaaaggt 7903
ggaatgagat ccaatttttc cccctaatcc ttcagtttaa gctgatacat gtaggttaat 7963
gtggaatgaa atcatctgtg atatattatg ttcatttatc aactgagctt ttttgatgtt 8023
gcctgttttt atgtaaaaca tgttcttaaa gttaataaaa taatagtact tggtgtaaa 8082

<210> 2
<211> 729

<212>  PRT
<213>  human

<400>  2

Met Ser Phe Gly Arg Asp Met Glu Leu Glu His Phe Asp Glu Arg Asp
1               5                   10                  15

Lys Ala Gln Arg Tyr Ser Arg Gly Ser Arg Val Asn Gly Leu Pro Ser
            20                  25                  30

Pro Thr His Ser Ala His Cys Ser Phe Tyr Arg Thr Arg Thr Leu Gln
        35                  40                  45

Thr Leu Ser Ser Glu Lys Lys Ala Lys Lys Val Arg Phe Tyr Arg Asn
    50                  55                  60

Gly Asp Arg Tyr Phe Lys Gly Ile Val Tyr Ala Ile Ser Pro Asp Arg
65                  70                  75                  80

Phe Arg Ser Phe Glu Ala Leu Leu Ala Asp Leu Thr Arg Thr Leu Ser
                85                  90                  95

Asp Asn Val Asn Leu Pro Gln Gly Val Arg Thr Ile Tyr Thr Ile Asp
            100                 105                 110

Gly Leu Lys Lys Ile Ser Ser Leu Asp Gln Leu Val Glu Gly Glu Ser
        115                 120                 125

Tyr Val Cys Gly Ser Ile Glu Pro Phe Lys Lys Leu Glu Tyr Thr Lys
    130                 135                 140

Asn Val Asn Pro Asn Trp Ser Val Asn Val Lys Thr Thr Ser Ala Ser
145                 150                 155                 160

Arg Ala Val Ser Ser Leu Ala Thr Ala Lys Gly Ser Pro Ser Glu Val
                165                 170                 175

Arg Glu Asn Lys Asp Phe Ile Arg Pro Lys Leu Val Thr Ile Ile Arg
            180                 185                 190

Ser Gly Val Lys Pro Arg Lys Ala Val Arg Ile Leu Leu Asn Lys Lys
        195                 200                 205

Thr Ala His Ser Phe Glu Gln Val Leu Thr Asp Ile Thr Asp Ala Ile
    210                 215                 220

Lys Leu Asp Ser Gly Val Val Lys Arg Leu Tyr Thr Leu Asp Gly Lys
225                 230                 235                 240

Gln Val Met Cys Leu Gln Asp Phe Phe Gly Asp Asp Asp Ile Phe Ile
                245                 250                 255

Ala Cys Gly Pro Glu Lys Phe Arg Tyr Gln Asp Asp Phe Leu Leu Asp
            260                 265                 270

Glu Ser Glu Cys Arg Val Val Lys Ser Thr Ser Tyr Thr Lys Ile Ala
        275                 280                 285

Ser Ser Ser Arg Arg Ser Thr Thr Lys Ser Pro Gly Pro Ser Arg Arg
    290                 295                 300

Ser Lys Ser Pro Ala Ser Thr Ser Ser Val Asn Gly Thr Pro Gly Ser
305                 310                 315                 320

Gln Leu Ser Thr Pro Arg Ser Gly Lys Ser Pro Ser Pro Ser Pro Thr
                325                 330                 335

Ser Pro Gly Ser Leu Arg Lys Gln Arg Ser Ser Gln His Gly Gly Ser
            340                 345                 350

Ser Thr Ser Leu Ala Ser Thr Lys Val Cys Ser Ser Met Asp Glu Asn
        355                 360                 365

Asp Gly Pro Gly Glu Glu Val Ser Glu Glu Gly Phe Gln Ile Pro Ala
    370                 375                 380

Thr Ile Thr Glu Arg Tyr Lys Val Gly Arg Thr Ile Gly Asp Gly Asn
385                 390                 395                 400

Phe Ala Val Val Lys Glu Cys Val Glu Arg Ser Thr Ala Arg Glu Tyr
                405                 410                 415

Ala Leu Lys Ile Ile Lys Lys Ser Lys Cys Arg Gly Lys Glu His Met
            420                 425                 430

Ile Gln Asn Glu Val Ser Ile Leu Arg Arg Val Lys His Pro Asn Ile
        435                 440                 445

Val Leu Leu Ile Glu Glu Met Asp Val Pro Thr Glu Leu Tyr Leu Val
    450                 455                 460

Met Glu Leu Val Lys Gly Gly Asp Leu Phe Asp Ala Ile Thr Ser Thr
465                 470                 475                 480

Asn Lys Tyr Thr Glu Arg Asp Ala Ser Gly Met Leu Tyr Asn Leu Ala
                485                 490                 495

Ser Ala Ile Lys Tyr Leu His Ser Leu Asn Ile Val His Arg Asp Ile
            500                 505                 510

Lys Pro Glu Asn Leu Leu Val Tyr Glu His Gln Asp Gly Ser Lys Ser
        515                 520                 525

Leu Lys Leu Gly Asp Phe Gly Leu Ala Thr Ile Val Asp Gly Pro Leu
    530                 535                 540

Tyr Thr Val Cys Gly Thr Pro Thr Tyr Val Ala Pro Glu Ile Ile Ala
545                 550                 555                 560

Glu Thr Gly Tyr Gly Leu Lys Val Asp Ile Trp Ala Ala Gly Val Ile
                565                 570                 575

Thr Tyr Ile Leu Leu Cys Gly Phe Pro Pro Phe Arg Gly Ser Gly Asp
            580                 585                 590

Asp Gln Glu Val Leu Phe Asp Gln Ile Leu Met Gly Gln Val Asp Phe
        595                 600                 605

Pro Ser Pro Tyr Trp Asp Asn Val Ser Asp Ser Ala Lys Glu Leu Ile
    610                 615                 620

Thr Met Met Leu Leu Val Asp Val Asp Gln Arg Phe Ser Ala Val Gln
625                 630                 635                 640

Val Leu Glu His Pro Trp Val Asn Asp Asp Gly Leu Pro Glu Asn Glu
                645                 650                 655

His Gln Leu Ser Val Ala Gly Lys Ile Lys Lys His Phe Asn Thr Gly
            660                 665                 670

Pro Lys Pro Asn Ser Thr Ala Ala Gly Val Ser Val Ile Ala Leu Asp
        675                 680                 685

His Gly Phe Thr Ile Lys Arg Ser Gly Ser Leu Asp Tyr Tyr Gln Gln
    690                 695                 700

Pro Gly Met Tyr Trp Ile Arg Pro Pro Leu Leu Ile Arg Arg Gly Arg
705                 710                 715                 720

Phe Ser Asp Glu Asp Ala Thr Arg Met
                725

<210>  3
<211>  26
<212>  DNA
<213>  artificial

<220>
<223>  primer

<400>  3
ggatgacttg gattcagtag gagact                                          26

<210>  4
<211>  30
<212>  DNA

<213>  artificial

<220>
<223>  primer

<400>  4
catggttagt gtgttcttgt actcaatatt                                        30


<210>  5
<211>  21
<212>  DNA
<213>  artificial

<220>
<223>  primer

<400>  5
ggagtggtga aacgcctgta c                                                 21


<210>  6
<211>  20
<212>  DNA
<213>  artificial

<220>
<223>  primer

<400>  6
ggttccatta actgagctgg                                                   20


<210>  7
<211>  29
<212>  DNA
<213>  artificial

<220>
<223>  primer

<400>  7
acactaagac tgtgtccatg ttagaactc                                         29


<210>  8
<211>  21
<212>  DNA
<213>  artificial

<220>
<223>  primer

<400>  8
aagccttcct ccgacacttc t                                                 21


<210>  9
<211>  31
<212>  DNA
<213>  artificial

<220>
<223>  primer

<400>  9
ctagactcga gcctcctgaa gatagctttg c                                      31

<210>  10
<211>  31
<212>  DNA
<213>  artificial

<220>
<223>  primer

<400>  10
cagacaagct tcagtctcag gaataccttg c                                    31


<210>  11
<211>  33
<212>  DNA
<213>  artificial

<220>
<223>  primer

<400>  11
gtcacggatc cttggaaact caagaagata ggc                                  33


<210>  12
<211>  31
<212>  DNA
<213>  artificial

<220>
<223>  primer

<400>  12
gatcagtcga cccacaggaa acaaagcaac c                                    31


<210>  13
<211>  201
<212>  DNA
<213>  human


<220>
<221>  polymorphism
<222>  (101)..(101)
<223>  a or c

<400>  13
tctacccttt agacctgtgg agaacaaatc aaatggtcct ggcttttgct tgacagtctc     60

attactttct cccaaatttg actcctcatt aactaggaat mctcagccct tcaactgctt    120

ctcatttgac atctttggaa atgctcacag cagtgcaact gtcagtgtct ccctccacat    180

ctgcctcccg gaactgggca t                                              201


<210>  14
<211>  201
<212>  DNA
<213>  human


<220>
<221>  polymorphsim
<222>  (101)..(101)
<223>  a or g

<400>  14
taccaatgtt tatggagtat ttaagtaaaa ttcatgtttg agtccatatg attaatgaaa      60

aacactaaac aggctatttt aagaaaatgt gatgtaatcc dtatagagac aattcaacgt     120

gcctttccac tagttccaag agctatagtt gagtagggac aggagacctg ggccagagca     180

gtggaggggc cctgagagag g                                               201

<210>  15
<211>  201
<212>  DNA
<213>  human

<400>  15
ctttaaacac aacaattggg agcagggatg ctcgcctgta atgattgttt gtgttagaaa      60

gttggctgcc accatggtta gacaaatgct cccacttctg yggagataaa aggcaagcac     120

tcgcaatcag taaggagagc gtgtacgttc tggggctaaa tggcattata aaacttccta     180

attaagcata tatgccgatg a                                               201

<210>  16
<211>  201
<212>  DNA
<213>  human

<400>  16
caacaagaac catgtaaaat catgggcatg atcaatttgg ggggaaaaac tttagttaaa      60

tacaacaata aatttataag ggtatttttt tattgggtca rtgcccagtt ttcacctaaa     120

atgtggctga aaacatccct gtaagtcaaa tcatattttc tcatcaaaat actttaaccc     180

aaccaaacac tagagttaaa t                                               201

<210>  17
<211>  201
<212>  DNA
<213>  human

<400>  17
ctttgtgttt cttttgacct cttgaacact cttatctcat aagaacaaat tttatacata      60

ctttgcctcg acatttgctt ttcttgataa ttttcagagc rtactctcta gcagtcgatc     120

tgcgaagaga aagttcatgt ttttaaaatg aaacttaact tcaaatctat tgaattattt     180

tgtcagggat caatttccta c                                               201

<210>  18
<211>  201
<212>  DNA
<213>  human

<400>  18
caggaatacc ttgctttaaa aagcaaaaca aaacaataaa agcctgtaag tatttagtaa      60

gtatccattg tatgtgtatt tttaaggcat tttttgaaag rcatttattt ttattgaagg     120

catttttatg caccagtcaa tgcacttaga ccttttatat atgaacatat atgcatctgt     180

gtgcatcagt atgtatatat a 201

<210> 19
<211> 201
<212> DNA
<213> human

<400> 19
tgaccttagg tgatccaccc aactcggcct cccaaagtgt tgggattaca ggtgtgagcc 60

accgcgccca gcctccaaga ctgtttaatt tctaaaagtg ycatgtgtct gacatatatt 120

tgagaattgt ttatttcagc ctatgaatac ttacaccctt tcttagcaaa aggtgtgttg 180

atacttataa ttacgttctt t 201

<210> 20
<211> 201
<212> DNA
<213> human

<400> 20
atgggtgtga atgaagaggc aggacaggtc actgtcccca ccatgggtcc atccctgccc 60

ctgaggtttg ttttaaggcc cggagatggt tcggtcttcc mgagtgcttc ctggttacag 120

gaatgagagc ctcccagggg cagggcagag aatcctggag aaccaaatac agtaacaaga 180

gcctagagct tagctttggt c 201

<210> 21
<211> 201
<212> DNA
<213> human

<400> 21
ataacatcaa caagatctca ttataattca tgcgcattac tacatggatt ctgggatatc 60

tcacctcaaa tcatctaaga tattactcgc ttatccagaa yagcctacga gaagcaaagt 120

ttaattctct actgtaccac ttacagctac tactttttaa aacagctcta ctgaggtata 180

acttacatgc cataaaattc a 201

<210> 22
<211> 201
<212> DNA
<213> human

<400> 22
caaaccaaac atgagaaatt atataattag cagcagcaca tttaaaagac gatataaaat 60

atgctaatgt tctcaccacg tgaaggcaaa gctgcagaaa ygatgccata tattagctag 120

cctaggatca gtcgaaatac agaaaaatct cttaagagca ttttaaaaat aatgatatca 180

atcagtttga tagaatgcct t 201

<210> 23
<211> 201
<212> DNA
<213> human

<400>  23
atcccttgct ctctggtcat caagtggacc agttcagaaa ccagtcagga atgaggagtg      60

ccagtaggtc atgtaaaaaa catgaaaagt taaacttggc ytgtaagccc agtgacatgt     120

caacaagcca ttcacaacaa tgatacatca gccaatgaga agtgatgcta cagaggctaa     180

aacacatttc actccacccc a                                               201

<210>  24
<211>  201
<212>  DNA
<213>  human

<400>  24
gttacatagg tgctccttgt ttctctcccc ttgctgggtt ttcatcaggt tttcttctga      60

tgaaacatat tgagagagga atgctctgtt gtaagaagac yctgagaggc gacaggatta     120

agccacacaa tacctctcag caagattttt gcagaaaagc atattgaagt gtgggcattt     180

caggagaagc cacaaaacca c                                               201

<210>  25
<211>  201
<212>  DNA
<213>  human

<400>  25
ggtaggaaca tgaaaggaga gccagatgca gtcaacagag caaacagatt gatgacttgg      60

gggaatgcag taagaccacc acctcccaag ggccatacac stttctacac atgtccctgt     120

tctgtggttg ctttaacaaa tcataacaca tggctagctt attctgtatc aaaacctgca     180

ttattttcca tgaattcaga a                                               201

<210>  26
<211>  201
<212>  DNA
<213>  human

<400>  26
tgtaatttgc tttagtaaag gggaactctg cacagcccac tcccctgctg gacagctcct      60

caaattcaac taagatccac ttccctggaa ttttcctgtg yaccctgggt ctacccttta     120

gacctgtgga gaacaaatca aatggtcctg gcttttgctt gacagtctca ttactttctc     180

ccaaatttga ctcctcatta a                                               201

<210>  27
<211>  201
<212>  DNA
<213>  human

<400>  27
tgcagctgtg aaacttcaag ataaacacag ttttcttgta agattaagaa aacaatataa      60

agccaactga aattttatca gtctttatta aaaccaaagt ratgcatatt ttattcctat     120

ttttagagtt attttattat aatttccctg tgtagataaa aaaaattgct taaaagaatt     180

ttacttttgc cactgcactc c                                               201

<210>  28
<211>  201
<212>  DNA
<213>  human

<400>  28
gggttatgtt tttctcttag caaacaatag gccttacagt ctatggacca aacatgtttc      60

tgttccgcaa tgcttgcagg attcacaaga gatgtactca ytttcattgt gaggcaatgg     120

tttctgtcat ggcagtaatt attggattta agtaactgca tttttcagag gcaaacattg     180

actgcttttc ctttttgct t                                                201

SEQUENCE LISTING

<110> Bergen Teknologioverforing AS

<120> DCLK1 and cognitive ability and cognitive performance

<130> 4200-007 EP-1

<160> 12

<170> PatentIn version 3.3

<210> 1
<211> 8082
<212> DNA
<213> human

<220>
<221> CDS
<222> (234)..(2423)

<400> 1
tttcaatgag gacgggccga ggcacatccc tgcactagtg gccgcaaccg aggcgccgcg 60

ctccagcagc tgctgccgcc cagcccggcc ccgccgccgc cccccagccc tgcagccccg 120

cagccccggc cgcgcccagc ccggcgagga cagcaccagg aggcggcccc cagcgcggcc 180

acaaagaccc ccggcggcgt ctctccgcgg accggtccta cttgaagtcc atc atg 236
Met
1

tcc ttc ggc aga gac atg gag ctg gag cac ttc gac gag cgg gat aag 284
Ser Phe Gly Arg Asp Met Glu Leu Glu His Phe Asp Glu Arg Asp Lys
            5                   10                  15

gcg cag aga tac agc cga ggg tcg cgg gtg aac ggc ctg ccg agc ccg 332
Ala Gln Arg Tyr Ser Arg Gly Ser Arg Val Asn Gly Leu Pro Ser Pro
        20                  25                  30

acg cac agc gcc cac tgc agc ttc tac cgc acc cgc acg ctg cag acg 380
Thr His Ser Ala His Cys Ser Phe Tyr Arg Thr Arg Thr Leu Gln Thr
    35                  40                  45

ctc agc tcc gag aag aag gcc aag aaa gtt cgt ttc tat cga aac gga 428
Leu Ser Ser Glu Lys Lys Ala Lys Lys Val Arg Phe Tyr Arg Asn Gly
50                  55                  60                  65

gat cga tac ttc aaa ggg att gtg tat gcc atc tcc cca gac cgg ttc 476
Asp Arg Tyr Phe Lys Gly Ile Val Tyr Ala Ile Ser Pro Asp Arg Phe
                70                  75                  80

cga tct ttt gag gcc ctg ctg gct gat ttg acc cga act ctg tcg gat 524
Arg Ser Phe Glu Ala Leu Leu Ala Asp Leu Thr Arg Thr Leu Ser Asp
            85                  90                  95

aac gtg aat ttg ccc cag gga gtg aga aca atc tac acc att gat ggg 572
Asn Val Asn Leu Pro Gln Gly Val Arg Thr Ile Tyr Thr Ile Asp Gly
        100                 105                 110

ctc aag aag att tcc agc ctg gac caa ctg gtg gaa gga gag agt tat 620
Leu Lys Lys Ile Ser Ser Leu Asp Gln Leu Val Glu Gly Glu Ser Tyr
    115                 120                 125

gta tgt ggc tcc ata gag ccc ttc aag aaa ctg gag tac acc aag aat 668
Val Cys Gly Ser Ile Glu Pro Phe Lys Lys Leu Glu Tyr Thr Lys Asn
130                 135                 140                 145

gtg aac ccc aac tgg tcg gtg aac gtc aag acc acc tcg gct tct cgg 716
Val Asn Pro Asn Trp Ser Val Asn Val Lys Thr Thr Ser Ala Ser Arg
                150                 155                 160

gca gtg tct tca ctg gcc act gcc aaa gga agc cct tca gag gtg cga 764
Ala Val Ser Ser Leu Ala Thr Ala Lys Gly Ser Pro Ser Glu Val Arg
            165                 170                 175

gag aat aag gat ttc att cgg ccc aag ctg gtc acc atc atc aga agt 812
Glu Asn Lys Asp Phe Ile Arg Pro Lys Leu Val Thr Ile Ile Arg Ser
        180                 185                 190

ggc gtg aag cca cgg aaa gct gtc agg att ctg ctg aac aag aaa acg 860
Gly Val Lys Pro Arg Lys Ala Val Arg Ile Leu Leu Asn Lys Lys Thr
    195                 200                 205

gct cat tcc ttt gag cag gtc ctc acc gat atc acc gat gcc atc aag 908
Ala His Ser Phe Glu Gln Val Leu Thr Asp Ile Thr Asp Ala Ile Lys
210                 215                 220                 225

ctg gac tcg gga gtg gtg aaa cgc ctg tac acg ttg gat ggg aaa cag 956
Leu Asp Ser Gly Val Val Lys Arg Leu Tyr Thr Leu Asp Gly Lys Gln
                230                 235                 240

gtg atg tgc ctt cag gac ttt ttt ggt gat gat gac att ttt att gca 1004
Val Met Cys Leu Gln Asp Phe Phe Gly Asp Asp Asp Ile Phe Ile Ala
            245                 250                 255

tgt gga ccg gag aag ttc cgt tac cag gat gat ttc ttg cta gat gaa 1052
Cys Gly Pro Glu Lys Phe Arg Tyr Gln Asp Asp Phe Leu Leu Asp Glu
        260                 265                 270

agt gaa tgt cga gtg gta aag tcc act tct tac acc aaa ata gct tca 1100
Ser Glu Cys Arg Val Val Lys Ser Thr Ser Tyr Thr Lys Ile Ala Ser
    275                 280                 285

tca tcc cgc agg agc acc acc aag agc cca gga ccg tcc agg cgt agc 1148
Ser Ser Arg Arg Ser Thr Thr Lys Ser Pro Gly Pro Ser Arg Arg Ser
290                 295                 300                 305

aag tcc cct gcc tcc acc agc tca gtt aat gga acc cct ggt agt cag 1196
Lys Ser Pro Ala Ser Thr Ser Ser Val Asn Gly Thr Pro Gly Ser Gln
                310                 315                 320

ctc tct act ccg cgc tca ggc aag tcg cca agc cca tca ccc acc agc 1244
Leu Ser Thr Pro Arg Ser Gly Lys Ser Pro Ser Pro Ser Pro Thr Ser
            325                 330                 335

cca gga agc ctg cgg aag cag agg agc tct cag cat ggc ggc tcc tct 1292
Pro Gly Ser Leu Arg Lys Gln Arg Ser Ser Gln His Gly Gly Ser Ser
        340                 345                 350

acg tca ctt gcg tcc acc aaa gtc tgc agc tcg atg gat gag aac gat 1340
Thr Ser Leu Ala Ser Thr Lys Val Cys Ser Ser Met Asp Glu Asn Asp
    355                 360                 365

ggc cct gga gaa gaa gtg tcg gag gaa ggc ttc cag att cca gct aca 1388
Gly Pro Gly Glu Glu Val Ser Glu Glu Gly Phe Gln Ile Pro Ala Thr
370                 375                 380                 385

ata aca gaa cga tat aaa gtc gga aga aca ata gga gat gga aat ttt 1436
Ile Thr Glu Arg Tyr Lys Val Gly Arg Thr Ile Gly Asp Gly Asn Phe
                390                 395                 400

gct gtt gtc aag gaa tgt gta gaa aga tcg act gct aga gag tat gct 1484
Ala Val Val Lys Glu Cys Val Glu Arg Ser Thr Ala Arg Glu Tyr Ala
            405                 410                 415

ctg aaa att atc aag aaa agc aaa tgt cga ggc aaa gag cac atg atc 1532
Leu Lys Ile Ile Lys Lys Ser Lys Cys Arg Gly Lys Glu His Met Ile
        420                 425                 430

cag aat gaa gtg tct att tta aga aga gtg aag cat ccc aat atc gtt 1580
Gln Asn Glu Val Ser Ile Leu Arg Arg Val Lys His Pro Asn Ile Val
    435                 440                 445

ctt ctg att gag gag atg gat gtg cca act gaa ctg tat ctt gtc atg 1628
Leu Leu Ile Glu Glu Met Asp Val Pro Thr Glu Leu Tyr Leu Val Met
450                 455                 460                 465

gaa tta gta aag ggg gga gac ctt ttt gat gcc att act tcc act aac 1676
Glu Leu Val Lys Gly Gly Asp Leu Phe Asp Ala Ile Thr Ser Thr Asn
                470                 475                 480

aaa tac acc gag aga gac gcc agt ggg atg ctg tac aac cta gcc agc 1724
Lys Tyr Thr Glu Arg Asp Ala Ser Gly Met Leu Tyr Asn Leu Ala Ser
            485                 490                 495

gcc atc aaa tac ctg cat agc ctg aac atc gtc cac cgt gat atc aag 1772
Ala Ile Lys Tyr Leu His Ser Leu Asn Ile Val His Arg Asp Ile Lys
        500                 505                 510

cca gag aac ctg ctg gtg tat gag cac caa gat ggc agc aaa tca ctg 1820
Pro Glu Asn Leu Leu Val Tyr Glu His Gln Asp Gly Ser Lys Ser Leu
    515                 520                 525

aag ctg ggt gac ttt gga ctg gcc acc att gta gac ggc ccc ctg tac 1868
Lys Leu Gly Asp Phe Gly Leu Ala Thr Ile Val Asp Gly Pro Leu Tyr
530                 535                 540                 545

aca gtc tgt ggc acc cca aca tac gtg gct cca gaa atc att gca gag 1916
Thr Val Cys Gly Thr Pro Thr Tyr Val Ala Pro Glu Ile Ile Ala Glu
                550                 555                 560

act gga tac ggc ctc aag gtg gac atc tgg gca gca ggt gta atc act 1964
Thr Gly Tyr Gly Leu Lys Val Asp Ile Trp Ala Ala Gly Val Ile Thr
            565                 570                 575

tat atc ctg ctg tgt ggt ttc cct cca ttc cgt gga agt ggt gat gac 2012
Tyr Ile Leu Leu Cys Gly Phe Pro Pro Phe Arg Gly Ser Gly Asp Asp
        580                 585                 590

cag gag gtg ctt ttt gat cag att ttg atg ggg cag gtg gac ttt cct 2060
Gln Glu Val Leu Phe Asp Gln Ile Leu Met Gly Gln Val Asp Phe Pro
    595                 600                 605

tct cca tac tgg gat aat gtt tcc gat tct gca aag gag ctc att acc 2108
Ser Pro Tyr Trp Asp Asn Val Ser Asp Ser Ala Lys Glu Leu Ile Thr
610                 615                 620                 625

atg atg ctg ttg gtc gat gta gat cag cga ttt tct gct gtt caa gta 2156
Met Met Leu Leu Val Asp Val Asp Gln Arg Phe Ser Ala Val Gln Val
                630                 635                 640

ctt gag cat ccc tgg gtt aat gat gat ggc ctc cca gaa aat gaa cat 2204
Leu Glu His Pro Trp Val Asn Asp Asp Gly Leu Pro Glu Asn Glu His
            645                 650                 655

cag ctg tca gta gct gga aag ata aag aag cat ttc aac aca ggc ccc 2252
Gln Leu Ser Val Ala Gly Lys Ile Lys Lys His Phe Asn Thr Gly Pro
        660                 665                 670

aag ccg aat agc aca gca gct gga gtt tct gtc ata gca ctg gac cac 2300
Lys Pro Asn Ser Thr Ala Ala Gly Val Ser Val Ile Ala Leu Asp His
    675                 680                 685

ggg ttt acc atc aag aga tca ggg tct ttg gac tac tac cag caa cca 2348
Gly Phe Thr Ile Lys Arg Ser Gly Ser Leu Asp Tyr Tyr Gln Gln Pro
690                 695                 700                 705

gga atg tat tgg ata aga cca ccg ctc ttg ata agg aga ggc agg ttt 2396
Gly Met Tyr Trp Ile Arg Pro Pro Leu Leu Ile Arg Arg Gly Arg Phe
                710                 715                 720

tcc gac gaa gac gca acc agg atg tga ggagccggta caaggcgcag 2443
Ser Asp Glu Asp Ala Thr Arg Met
            725

ccagctcctc ccgaactcaa ctcggaatcg gaagactact ccccaagctc ctccgagact 2503

gttcgctccc ctaactcgcc cttttaataa gaccctttta ctcaaagtcc tagcttaacc 2563

ctttgagact ctgagatttt tttcccccaa atttgtgtaa aacagtttca tctgatctat 2623

ctagcgctca atgcttgaat ggcagaactg aaagtgtttt caggtatctt tgtagcggtt 2683

tccctttact gaataagatg acacgtggtg attgtgaaga tggtaatttg ctgctaatag 2743

agtcctcaaa gggttaaggc caatttgcaa ttttttttta aacttagaag caatgaatgt 2803

tttcatcagt caagctagga tctgcagtat gtaatatagc acttgttaac cctctgagtg 2863

catagaattt tattgagaat tcttgtttgg gaatttttca ggcctttgga tgtatacaca 2923

catgtttctt gattttactg cagatcaagg ggtgttgtta gatgctgaaa tgtccagaaa 2983

agaaggacat ttagaatgat atcttgtttg tccttttctg tgggtttaga acgtggcagg 3043

tttataactt cgacacacgc acggttcttt cttcttcaca atcctattca gaaacagatt 3103

ttttttttca ttagagatat gactgtcagt tgcagtgagt tctgcatccc aagtggaggg 3163

aattgggttt gtggcaaaga gcttgaccca ggaaatagat ggtgcccccc aaattgtctc 3223

cacatgaaga tgtactgatg acgccccaga aatgctgctt ccatatcagc tgctgctagc 3283

gccagcgcag actctcaggg agtcaccaca gcttgtcttg tgcttggtga gtgagggtct 3343

ctctactcag tgtcagacat ctacaggaaa gaaacaactg gtggaaaaga gcaataaatt 3403

gcccggtgct ctgcagggct ggaatttcaa acagaaagag ggaataagat cctgtgattt 3463

ttctcacctg cttttccacg cactgtggtc atcactgtgc aatctacatc tagtatgaaa 3523

tccacacata ggagagctgg ggcacaaggg gactggaggc agttgctttg caagatggct 3583

gaggagaaag cacactggga acacaatcca gaatgttcta acaataagtt ttcagtgaat 3643

aaaccactgg caagacaatt ccatgtgcac ctttaggtta cctatatagt ctcctaggaa 3703

gatcaggatg aaagacctag atgatacccc tgaggataaa acctccatcc cctaaaatga 3763

ttttttttaa ataccactgt ctttagctgt ccaggaggtc agagtgtttt ttctgtcttt 3823

gggccaagtc ctgtctgaga cctgtatttt cactcttgtt accaaatcta tctccctagt 3883

gcagtgtctc caggcctgag tttcttctgg aacagattcc attttagaat ggggattcac 3943

aggttctgtg catcaccaca gtgctcagag aggattctcc tggggtgtct tagaggcagg 4003

tgcccaactc aaatgtattc ccaaggtttg ctgggctctg ggatccacga gacaaccaga 4063

gagggatatc tcatgaaatt tgcatctggt ggctgaacag tacctatgtt ctctgttttg 4123

aatatacttt aatacctgag agtcttaaaa tttgtgaaca acgtttctat agtcctttat 4183
tttcaaatgc acattgatct tcacttgctg catttttact cttcaaccct gaaactatgg 4243
tctacattaa tatggatttt taaatcacat gtcattactt ttgcaacacc atcaccaaaa 4303
ttttttgctc ttttacattt aggttcatct ctgtggtctg tgttgtcctg acatgtaaaa 4363
agcatatcgt ttattgaggt ttttttcccc cccttttaga gcatccggaa gtgataacac 4423
gcaaaatcac aaagtagcat aaatcagtaa attagttgag ttgtttttgg gggggaggtg 4483
ggggtagggg gcacagaaca ccagaaagag tgttggtgtg taggtagatt ccatattaat 4543
gaggaacact gaactagttg gaaattactg ctttctctag aaatataaag caaagcacta 4603
ttccaaggct atggagtagc tctacagcct ggcctcaact ctaaaagtgt gaagaatgca 4663
atgggcagag acctacctgc agtggactgt cattttcctt tctttctctg aattactgct 4723
ttttctgtgg gcattaacta tattgctaca gcatctagtg tactgagcct gcggtgcatg 4783
gctcaggcct tttcccatcg acgtctaggg ggactctgga ccgtgtgaag ctagggggtg 4843
tttctcagca cactgcagaa gggcagctca gaagaatgca gggcccattc agcatgggga 4903
tcccagcaca tcactgtaga atttgagtga tctatgctga ataaacagtg gaatgtgacc 4963
agtcaagtag aaatcttgag taatcagatg gaatgcaatc tttctaacat taagctacca 5023
agatcctgaa tgtcagagat gtactcagag ggttaacaga caagcacaag gcatgctgac 5083
tacattggtg tatccagatt gctttgcttt tagccagtgc tttctaattt ttttctcgac 5143
attcttggga tagttcaagt ttgaaataat taagtggtgg tgttctttaa ggaatttcta 5203
taaccaaatt gatcttattt ttgatttcac ttatcataga acaaatatgt atcattatgg 5263
cagtgtatct atgtaattat caatttaatc atcaccaccg gtgtttccat attttttccc 5323
aagtatttaa tatagctctc ttatggtggt ggcctggtga tggggaccgt ctttctttta 5383
ctgacacatg accaatcata tggtattttc aagggaattt taagattcat cttttcagtt 5443
tgatagtaga ctagttaagg aagaactctt tcattacttg catcgtgtaa atcatctctg 5503
tagacatgtg ttcatattaa tgaacacatt ttttctcaac attgtagcag aaatcatttt 5563
attcgtcatg atcaatgaat atgtgatttg ctccagatcg ttagaaggaa aagtaagatt 5623
tcagtcatca aaaatgtttt taccgtagcc ctcatctaac ttacacgtgg tgcatattaa 5683
aataagcaga gaaaaaaaaa tgtgaataaa ctactgaaaa cacttggtgt tttgtgttca 5743
atgagacctt cctgcaacct gctccccatg ggtggcagtt aacaggccca tcagatattg 5803
ttgaaagaaa gcaatatatc catgaatgaa ggctaaaatt gcaatccttt accctttgag 5863
gcatatttca gttgaaaaca aaaagaaaag aaaatttggc ttagagggtc acagagctcc 5923
catatgacca agtctcaagc acattaaatc atggttgttt actggccaag ggcgtccact 5983
agacaactct atcccttgcg ctgaagctca atcgtgctga gggagagctt tcttaatatt 6043
actgtgttgc tcttagccct tctctgggtt aggatctgtc agcatttcta tgataaactc 6103
ctattctcaa aggtttttaa tttgaccata aaaatgtgcc ccaggctgaa gtttgctata 6163

cagggctgta ccaaagagtg aaggtttact tccttctctt tccaacttct tccccattct 6223
ccaaggaaaa gaacaacaaa aaaatctggt atggtccctc cttaatagtg atttcagaat 6283
tttggaaagc accaagatcc aagatggtag ttttaatgta gttactcatt cgcacacatt 6343
ttttaaattt aatgggtcac ctggcatata ttgtagataa catatctttt ctataatttg 6403
taagtcaata atttttttaa ctgctacatg atattttttt ttgcccaaag attttaaaag 6463
acttgaagtt ggtcagttca aaactcagat ttttctacac attgtctgcc atgtccatta 6523
ggagtttggg gaaaatactc tcacacagac ccttactttg catgcagttt agagggtaag 6583
atacgtgctt cttttgggga taaagatttc cttacttaat tgtcaaattt catggagcca 6643
ttctagtctg ttggggaaaa tagtgattaa aagcacttcc aaaattaaca ttttttgaca 6703
attcagatat gaaaagaagc aggggaaaat aatacacttt actcttttct tgcttaaagg 6763
caaacaaatc aatgaaactt gaggacacac taaacatttg ataactgcaa atgtgcttta 6823
aaaattggtt caatggtgct tacacatgaa acggtaacaa atggggttcc taggacgtca 6883
gaaggaatct ttagtttgta tgtaattaca cactagagga ggaggtgctt ttaagccagt 6943
cttttatttt taatcatctc aaatatgcaa ccatacatgc agtaacatta agggtcgtaa 7003
actggtggga aacaggaact tcagtggaga ggcttaaatg cctctggtta gagtgggggt 7063
ttttgtttgt ttgtttattg ttgggtttca acactgagca tcatttctgt gatcaagttt 7123
ctaactggca tgtgttttga tcatgaggtt taccatatct tgcccataca gacaaatgag 7183
agatctagtt tcattttgtt ccctaaagaa agaacactct ctaaaattaa atcatacctg 7243
taaatttctt cagcatttgt ttctgttcaa tgaaattgag acccttaatg ttgctttaat 7303
gtaaaattga atattttgtc tgtgatatac tttaataatt taaagtaagt aatagttcta 7363
aagtcttcac tgttgctact aagagaaaat agaattttaa aggtgatgat aaagatgcta 7423
taatgtcagt tcactccagt ccaatcaaat gtagtaagaa aaagtccttg aatagttctc 7483
tagggacaat ttctcacttg ccattgacat taatctttgg tgtattctca gaaaaaataa 7543
aaagaaattg aaactggtcc aaggttatag tcatatcctc gataactttt gaaaaaaaat 7603
tttattagga aattaatact agcctttttc attctggctg aaagaaaatt attaaaggat 7663
tagttgagtg tgaaattcaa cagtattttg ctcatacata ctaaaaaggt gcgtagggac 7723
ttggcgcatt taaacaagtt tctgaaaggt ttcaatttga ctcaagaaaa aaattcaata 7783
tttcttttga aaatactgaa tttatcactt gctgcatgga tcagatggca taggttaatc 7843
tttgattttc agaatcctaa tgaaataact ttcaaacaat ttgtgtcctt aattaaaggt 7903
ggaatgagat ccaatttttc cccctaatcc ttcagtttaa gctgatacat gtaggttaat 7963
gtggaatgaa atcatctgtg atatattatg ttcatttatc aactgagctt ttttgatgtt 8023
gcctgttttt atgtaaaaca tgttcttaaa gttaataaaa taatagtact tggtgtaaa 8082

<210> 2
<211> 729

<212>  PRT
<213>  human

<400>  2

Met Ser Phe Gly Arg Asp Met Glu Leu Glu His Phe Asp Glu Arg Asp
1               5                   10                  15

Lys Ala Gln Arg Tyr Ser Arg Gly Ser Arg Val Asn Gly Leu Pro Ser
            20                  25                  30

Pro Thr His Ser Ala His Cys Ser Phe Tyr Arg Thr Arg Thr Leu Gln
        35                  40                  45

Thr Leu Ser Ser Glu Lys Lys Ala Lys Lys Val Arg Phe Tyr Arg Asn
    50                  55                  60

Gly Asp Arg Tyr Phe Lys Gly Ile Val Tyr Ala Ile Ser Pro Asp Arg
65                  70                  75                  80

Phe Arg Ser Phe Glu Ala Leu Leu Ala Asp Leu Thr Arg Thr Leu Ser
                85                  90                  95

Asp Asn Val Asn Leu Pro Gln Gly Val Arg Thr Ile Tyr Thr Ile Asp
            100                 105                 110

Gly Leu Lys Lys Ile Ser Ser Leu Asp Gln Leu Val Glu Gly Glu Ser
        115                 120                 125

Tyr Val Cys Gly Ser Ile Glu Pro Phe Lys Lys Leu Glu Tyr Thr Lys
    130                 135                 140

Asn Val Asn Pro Asn Trp Ser Val Asn Val Lys Thr Thr Ser Ala Ser
145                 150                 155                 160

Arg Ala Val Ser Ser Leu Ala Thr Ala Lys Gly Ser Pro Ser Glu Val
                165                 170                 175

Arg Glu Asn Lys Asp Phe Ile Arg Pro Lys Leu Val Thr Ile Ile Arg
            180                 185                 190

Ser Gly Val Lys Pro Arg Lys Ala Val Arg Ile Leu Leu Asn Lys Lys
        195                 200                 205

Thr Ala His Ser Phe Glu Gln Val Leu Thr Asp Ile Thr Asp Ala Ile
    210                 215                 220

Lys Leu Asp Ser Gly Val Val Lys Arg Leu Tyr Thr Leu Asp Gly Lys
225                 230                 235                 240

Gln Val Met Cys Leu Gln Asp Phe Phe Gly Asp Asp Asp Ile Phe Ile
                245                 250                 255

Ala Cys Gly Pro Glu Lys Phe Arg Tyr Gln Asp Asp Phe Leu Leu Asp
            260                 265                 270

Glu Ser Glu Cys Arg Val Val Lys Ser Thr Ser Tyr Thr Lys Ile Ala
        275                 280                 285

Ser Ser Ser Arg Arg Ser Thr Thr Lys Ser Pro Gly Pro Ser Arg Arg
    290                 295                 300

Ser Lys Ser Pro Ala Ser Thr Ser Ser Val Asn Gly Thr Pro Gly Ser
305                 310                 315                 320

Gln Leu Ser Thr Pro Arg Ser Gly Lys Ser Pro Ser Pro Ser Pro Thr
                325                 330                 335

Ser Pro Gly Ser Leu Arg Lys Gln Arg Ser Ser Gln His Gly Gly Ser
            340                 345                 350

Ser Thr Ser Leu Ala Ser Thr Lys Val Cys Ser Ser Met Asp Glu Asn
        355                 360                 365

Asp Gly Pro Gly Glu Glu Val Ser Glu Glu Gly Phe Gln Ile Pro Ala
    370                 375                 380

Thr Ile Thr Glu Arg Tyr Lys Val Gly Arg Thr Ile Gly Asp Gly Asn
385                 390                 395                 400

Phe Ala Val Val Lys Glu Cys Val Glu Arg Ser Thr Ala Arg Glu Tyr
                405                 410                 415

Ala Leu Lys Ile Ile Lys Lys Ser Lys Cys Arg Gly Lys Glu His Met
            420                 425                 430

Ile Gln Asn Glu Val Ser Ile Leu Arg Arg Val Lys His Pro Asn Ile
        435                 440                 445

Val Leu Leu Ile Glu Glu Met Asp Val Pro Thr Glu Leu Tyr Leu Val
    450                 455                 460

Met Glu Leu Val Lys Gly Gly Asp Leu Phe Asp Ala Ile Thr Ser Thr
465                 470                 475                 480

Asn Lys Tyr Thr Glu Arg Asp Ala Ser Gly Met Leu Tyr Asn Leu Ala
                485                 490                 495

Ser Ala Ile Lys Tyr Leu His Ser Leu Asn Ile Val His Arg Asp Ile
            500                 505                 510

Lys Pro Glu Asn Leu Leu Val Tyr Glu His Gln Asp Gly Ser Lys Ser
        515                 520                 525

Leu Lys Leu Gly Asp Phe Gly Leu Ala Thr Ile Val Asp Gly Pro Leu
    530                 535                 540

Tyr Thr Val Cys Gly Thr Pro Thr Tyr Val Ala Pro Glu Ile Ile Ala
545                 550                 555                 560

Glu Thr Gly Tyr Gly Leu Lys Val Asp Ile Trp Ala Ala Gly Val Ile
                565                 570                 575

Thr Tyr Ile Leu Leu Cys Gly Phe Pro Pro Phe Arg Gly Ser Gly Asp
            580                 585                 590

Asp Gln Glu Val Leu Phe Asp Gln Ile Leu Met Gly Gln Val Asp Phe
        595                 600                 605

Pro Ser Pro Tyr Trp Asp Asn Val Ser Asp Ser Ala Lys Glu Leu Ile
    610                 615                 620

Thr Met Met Leu Leu Val Asp Val Asp Gln Arg Phe Ser Ala Val Gln
625                 630                 635                 640

Val Leu Glu His Pro Trp Val Asn Asp Asp Gly Leu Pro Glu Asn Glu
                645                 650                 655

His Gln Leu Ser Val Ala Gly Lys Ile Lys Lys His Phe Asn Thr Gly
            660                 665                 670

Pro Lys Pro Asn Ser Thr Ala Ala Gly Val Ser Val Ile Ala Leu Asp
        675                 680                 685

His Gly Phe Thr Ile Lys Arg Ser Gly Ser Leu Asp Tyr Tyr Gln Gln
    690                 695                 700

Pro Gly Met Tyr Trp Ile Arg Pro Pro Leu Leu Ile Arg Arg Gly Arg
705                 710                 715                 720

Phe Ser Asp Glu Asp Ala Thr Arg Met
                725

<210>  3
<211>  26
<212>  DNA
<213>  artificial

<220>
<223>  primer

<400>  3
ggatgacttg gattcagtag gagact                                          26

<210>  4
<211>  30
<212>  DNA

<213>  artificial

<220>
<223>  primer

<400>  4
catggttagt gtgttcttgt actcaatatt                                        30


<210>  5
<211>  21
<212>  DNA
<213>  artificial

<220>
<223>  primer

<400>  5
ggagtggtga aacgcctgta c                                                 21


<210>  6
<211>  20
<212>  DNA
<213>  artificial

<220>
<223>  primer

<400>  6
ggttccatta actgagctgg                                                   20


<210>  7
<211>  29
<212>  DNA
<213>  artificial

<220>
<223>  primer

<400>  7
acactaagac tgtgtccatg ttagaactc                                         29


<210>  8
<211>  21
<212>  DNA
<213>  artificial

<220>
<223>  primer

<400>  8
aagccttcct ccgacacttc t                                                 21


<210>  9
<211>  31
<212>  DNA
<213>  artificial

<220>
<223>  primer

<400>  9
ctagactcga gcctcctgaa gatagctttg c                                      31

<210>  10
<211>  31
<212>  DNA
<213>  artificial

<220>
<223>  primer

<400>  10
cagacaagct tcagtctcag gaataccttg c                                    31


<210>  11
<211>  33
<212>  DNA
<213>  artificial

<220>
<223>  primer

<400>  11
gtcacggatc cttggaaact caagaagata ggc                                  33


<210>  12
<211>  31
<212>  DNA
<213>  artificial

<220>
<223>  primer

<400>  12
gatcagtcga cccacaggaa acaaagcaac c                                    31